# EUROPEAN PATENT SPECIFICATION

(11) **EP 3 280 999 B1**
(45) Date of publication and mention of the grant of the patent: **08.05.2024**
(21) Application number: 16702385.2
(22) Date of filing: 27.01.2016
(51) Int. Cl.: G01N 21/47, G03H 1/04

(54) **METHOD AND SYSTEM FOR IDENTIFYING MICROORGANISMS**
VERFAHREN UND SYSTEM ZUR IDENTIFIZIERUNG VON MIKROORGANISMEN
PROCÉDÉ ET SYSTÈME D'IDENTIFICATION DE MICRO-ORGANISMES

(30) Priority: 10.04.2015 PL 41194615
(43) Date of publication of application: 14.02.2018
(73) Proprietor: Bioavlee Sp. z o.o., 50-428 Wroclaw (PL)
(72) Inventor: ANDRZEJEWSKI, Damian, 50-428 Wroclaw (PL); BUZALEWICZ, Igor, 50-428 Wroclaw (PL); BEDNAREK, Karolina, 50-428 Wroclaw (PL); MARKIEWICZ, Natalia, 50-428 Wroclaw (PL); PAWELEK, Lukasz, 50-428 Wroclaw (PL)
(74) Representative: Witek, Rafal
(86) International application number: PCT/EP2016/051715
(87) International publication number: WO 2016/162132

(56) References cited:
- WO-A1-2013/138513
- WO-A1-2014/184390
- FR-A1- 3 001 544

## Description

### Field of the Invention

The present invention relates to a method and system for identifying microorganisms.

### Background to the Invention

Systems and methods for the rapid detection and characterization of bacterial colonies using light scattering are known for example from US 7465560. The system for characterizing bacterial colonies disclosed therein comprises a light source, a holder adapted for holding a solid medium having a surface adapted for the growth of a bacterial colony, a holder disposed in such a way that light emanating from the light source impinges upon at least one colony cultivated on the solid medium, and a film or an electronic optical sensor for obtaining an image. The method of rapid detection and characterization of bacterial colonies includes a bacterial colony on a solid medium being disposed between a laser and an optical detector. Light from the light source is directed at the bacterial colony and light scattered by the bacteria is detected by the optical detector. The signal is then analyzed by an analyzer and is displayed on a screen or saved on a storage medium.

A method of testing bacterial colonies cultivated on solid media and an optical system for testing bacterial colonies cultivated on solid media are known from PL 218010 B1. The method includes bacterial colonies being irradiated with a beam of laser light, which is then detected, and diffraction spectra are analyzed. A collimated beam of light from a coherent light source is filtered in an amplitude filter, polarized in a linear polarizer and expanded using a beam expander. The beam diameter is adjusted with an iris diaphragm and the beam converges in the rear focal plane of a positive lens. So formed, the converging spherical beam of light irradiates the bacterial colony being tested, which is disposed on a transparent medium placed on a transparent plate mounted on a stand between the lens and its rear focal plane. The beam is transmitted and diffracted through the bacterial colony, and the light is monitored by the detector. The two-dimensional distribution of luminous intensity of light diffracted through the bacterial colony is then recorded and analyzed in a computer. The optical system for testing bacterial colonies cultivated on solid media comprises a laser and a detector connected with an analyzing system comprising a screen and a storage medium. The system comprises a coherent light source generating a collimated beam of light along an optical axis of the system. Arranged along the optical axis are an amplitude filter, a linear polarizer, a beam expander, an iris diaphragm, and a positive lens. A bacterial colony to be tested is disposed on a transparent medium placed on a transparent plate mounted on a stand, and a detector connected to a computer is provided. WO2013/138513 A1 discloses a system and method for identifying viability and/or differentiation status of cell colonies by analysis of scattergrams of colonies. FR3001544A1 discloses a method and devices for recording diffraction patterns of cell cultures.

In the fields of bacteria and microorganism recognition, the speed and reliability with which species can be characterized is of high importance. Therefore, there exists a demand for more rapid, efficient and accurate diffraction analysis techniques for microbial identification.

### Summary of the Invention

In accordance with the invention there is provided a method of identifying microorganisms as defined in independent claim 1, the method comprising inter alia:
acquiring data representing a region on a plate within which at least one colony of microorganisms is disposed;
exposing a target colony of the at least one colony of microorganisms to a beam of coherent light, in accordance with the data;
monitoring a diffraction pattern resulting from the diffraction of the light by the target colony; and
generating target colony data in accordance with the monitored diffraction pattern and a reference diffraction pattern.

The method allows the rapid, efficient, accurate, non-destructive and contactless testing of bacterial colonies by using diffraction analysis in a manner that targets individual microbial colonies based upon data representing the distribution of colonies on a plate. The data may be acquired by one or more of a variety of methods, including: taking an image of the plate; scanning the plate with a radiation beam; inoculating one or more colonies in known or predetermined positions on the plate; and receiving data representing the distribution of microorganisms on the plate from a separate or remote system or monitoring device.

The data representing the region on the plate may typically comprise optical data representing an image of the region, and the position on the plate of the target colony of the at least one colony of microorganisms may be located in accordance with the optical data.

Typically, the target colony data includes data indicating a species present in the target colony. The method may thus allow the identification of microorganisms based upon the characteristic manner in which a colony of microorganisms behaves as an optical element and diffracts light that is incident upon it.

Preferably, the acquired data representing the region on the plate comprises an indication of the position on the plate of the target colony. Where the data comprises an image of a region on the plate, the colony position may be indicated by a visible optical pattern corresponding to the colony having different optical properties, such as a opacity, translucency or a colour that allows it to be distinguished visually from the surrounding plate and growth medium, each of which will typically be transparent.

In some embodiments, exposing the target colony of the at least one colony of microorganisms to the beam of coherent light in accordance with the data comprises: locating the position on the plate of the target colony in accordance with the data; and exposing the target colony to the beam of coherent light. Thus the data allows a position on the plate, or within the region on the plate, of the target colony to be calculated so that this colony may be targeted by the beam of coherent light for the purpose of diffraction analysis identification.

The target colony data is generated based upon the monitored diffraction pattern and a reference diffraction pattern. In practice, a computer may be used to rapidly compare the monitored pattern with a large number of reference diffraction patterns. Since different species (or different types, genera, families, orders, phyla or other taxonomic categories) of microorganisms may have different, characteristic optical and morphological properties, the diffraction pattern produced when a colony is irradiated may allow the species or type of microorganism present in the colony to be identified by recognizing a degree of similarity between the pattern and a reference pattern produced by a known species or type of microorganism. The target colony data is generated by comparing the monitored diffraction pattern with the reference diffraction pattern.

The method further comprises:
generating a comparison parameter based upon a comparison of the monitored diffraction pattern with each of a plurality of reference diffraction patterns, each reference diffraction pattern corresponding to a predetermined species; and identifying the target species present in the target colony as the predetermined species for which the generated comparison parameter has the greatest value.

Typically, exposing the target colony to the beam of coherent light comprises causing the beam of coherent light to impinge upon a portion of the region on the plate corresponding to the position on the plate of the target colony.

Typically a part of the region on the plate upon which the beam of coherent light impinges is adjusted by adjusting one or more of: the direction of the beam; the position of the source of the beam; and the position of the plate. Such movement is preferably effected by way of automated mechanisms in order that the method may be performed in a consistent, repeatable and efficient manner.

Typically, the method further comprises adjusting the point at which the central axis of the beam impinges upon the plate such that this point is centred upon the position on the plate of the target colony.

Typically, the method further comprises adjusting the relative positions of a source of the beam of coherent light and the plate such that the beam is directed towards the position on the plate of the target colony.

Preferably, exposing the target colony to the beam of coherent light comprises directing the beam towards an optical centroid of the target colony. The optical centroid corresponds to a point within the target colony, which may be the same as or different from the geometric centroid of the colony, that results in the highest quality diffraction pattern being produced when the beam of light is centred upon that point.

The method may further comprise generating a symmetry parameter for a point within the region on the plate based upon the symmetry of the monitored diffraction pattern produced when the beam of coherent light is centred upon that point, and; wherein the optical centroid is defined as a point within the target colony corresponding to the greatest value of the symmetry parameter.

Typically, the symmetry parameter for a point within the region on the plate is generated based upon any of the symmetry, contrast or homogeneity of the monitored diffraction pattern produced when the beam of coherent light is centred upon that point

Typically, directing the beam towards the optical centroid comprises adjusting the point at which the central axis of the beam of coherent light impinges upon the plate and monitoring the resulting diffraction pattern so as to ascertain a point within the target colony that corresponds to a maximum value of the symmetry parameter.

Typically, directing the beam towards the optical centroid comprises adjusting the relative positions of the plate and a source of the beam such that the beam coincides with the position on the plate of the target colony, and correcting the relative positions until the beam coincides with a point corresponding to a maximum value of the symmetry parameter.

Typically, directing the beam towards the optical centroid comprises iteratively adjusting the point within the region on the plate upon which the central axis of the beam impinges and generating a symmetry parameter, until a maximum value of the symmetry parameter is generated and a point corresponding to this value is located.

Typically, exposing the target colony to a beam of coherent light comprises adjusting an optical system comprising a source of the beam of coherent light relative to the plate such that the beam is directed towards the position on the plate of the target colony.

The method may comprise adjusting the optical system relative to the plate by moving the plate in any of a direction Z parallel to the optical axis of the system and directions X and Y perpendicular to the optical axis of the system.

Typically, acquiring the data comprises acquiring an image of the region on the plate. This may be performed with any imaging device suitable for recording optical data indicating the position or distribution of colonies on the plate. Illumination for recording the image may be provided by ambient light, coherent light from the source of the beam, or a separate light source capable of producing radiation of a suitable frequency and intensity for allowing the colonies to be visually distinguished on the plate.

Typically, acquiring the data comprises acquiring optical data by exposing the region on the plate to radiation and acquiring an image of the region and the at least one colony of microorganisms. The image may be recorded from a direction perpendicular to the surface region of the plate within which the at least one colony is disposed. The image may also be recorded from a direction parallel to the surface region of the plate within which the at least one colony is disposed.

Typically, the image is recorded from the direction parallel to the surface of the plate by recording the beam reflected from a light splitter.

The method may comprise locating the position on the plate of the target colony by analysing the acquired image using a computer configured to process image data so as to identify and locate the position of a colony of microorganisms within the image.

Typically, acquiring the data comprises causing the beam of coherent light to scan the region on the plate. The method may comprise locating the position on the plate of the target colony by monitoring light resulting from the beam of coherent light scanning the region on the plate and detecting light diffracted by the target colony.

In some embodiments, prior to the monitoring of the diffraction pattern of the target colony: the at least one colony of microorganisms disposed within the region on the plate is irradiated; an image of the entire plate and the at least one colony of microorganisms is recorded, sent to a computer, analysed using computer algorithms for processing visual information, and the location of the at least one colony of microorganisms recorded is distinguished and identified, and then: the plate and the at least one colony of microorganisms is moved relative to an optical system comprising a source of the coherent light beam; and a single colony of microorganisms of the at least one colony of microorganisms is irradiated with the coherent beam of light, and, subsequent to the monitoring of the diffraction pattern of the target colony, the pattern is recorded and compared with a reference pattern.

In some embodiments, after the location on the plate of the colonies of microorganisms is distinguished and identified, the plate is moved in relation to the optical system and an enlarged image of each of at least one individual colony is acquired.

Typically, algorithms for processing visual information comprise analysis of colony morphologies. The use of morphological data, that is data related to or indicating the physical or apparent shapes and geometric properties of microbial colonies, provides further insight into the properties of the target colony. Such data may be used in combination with diffraction analysis data in order to more accurately identify or characterize microorganisms within the target colony.

Typically, the beam of coherent light comprises a laser beam. Owing to the collimated, coherent and monochromatic nature of light beams produced by laser, diffraction patterns of a laser beam passed through objects are suitable for analysing or measuring the geometrical dimensions or properties of object or elements ranging from nanometres to millimetres in size or periodicity.

Each of the diffraction patterns, that is the monitored pattern for a colony, or a reference pattern, may comprise a diffraction spectrum.

In some embodiments, the step of acquiring the data may be repeated at least until the acquired data indicates the position on the plate of the target colony. The step may be repeated at a predetermined interval and over a sufficiently long period of time for a colony of microorganisms to grow on the plate, and the data indicating the position on the plate of the target colony may be defined by the target colony having grown to such a size that it may be visually located in accordance with the data.

In some embodiments, the steps of exposing the target colony to the beam, monitoring the diffraction pattern, and generating target colony data are repeated at least until the target colony data includes an identified species present in the target colony. The steps may be repeated according to a predetermined time interval and over a sufficiently long period of time for a colony of microorganisms to grow on the plate, and the target colony data may include an identified species present in the target colony defined by the target colony having grown to such a size that the diffraction pattern contains sufficient data to identify the species with reference to a reference diffraction pattern.

The target colony data may be generated in accordance with two or more monitored diffraction patterns, wherein the two or more patterns are monitored at different times and are representative of the diffractive properties of the target colony at the different times. The target colony data may be representative of a change in the diffractive properties of the target colony between the different times.

In some embodiments, the method further comprises: inoculating at least one microorganism onto the plate; and incubating the at least one microorganism so as to allow the development of at the least one colony of microorganisms disposed in the region on the plate.

Each of the inoculating and incubating may be performed by an automated system in accordance with predetermined inoculation parameters and incubation parameters, respectively. The inoculation and incubation parameters may be configured such that, each time the method is performed, the physical processes by way of which the colonies of microorganisms are inoculated and incubated are performed identically. Having the inoculation and incubation performed by an automated or robotic system, in a repeatable way, minimises the variation and improves the repeatability of the culturing of microbial colonies for analysis. In this way, superior analysis results are achieved, since the effects of any variations in the conditions in which microorganisms are disposed and developed will be mitigated. Additionally, the parameters may be tuned or optimised in order to more reliably or more efficiently prepare colonies that are optimised for diffraction or morphological analysis.

In some embodiments, the data representing the region on the plate is acquired in accordance with the inoculation parameters, such that the data includes an indication of the portion of the plate within which the at least one microorganism was disposed; and the position on the plate of the target colony is located in accordance with the indicated portion of the plate. This technique for locating target colonies may be used as an alternative to, or in combination with, data acquisition techniques involving optical data such as beam scanning or snapshot capturing.

The data representing the region on the plate may typically comprise optical data representing an image of the indicated portion; and the position on the plate of the target colony may be located in accordance with the optical data. Acquiring the optical data may comprise causing the beam of coherent light to scan the indicated portion of the plate; and locating the position of the target colony may comprise monitoring light resulting from the beam of coherent light scanning the portion of the plate and detecting light diffracted by the target colony.

Typically, the region on the plate comprises a culture medium, and the at least one colony of microorganisms is disposed on the medium. This growth medium or culture medium may comprise a liquid or gel suitable for supporting the growth of microorganisms.

Some embodiments of the method may comprise causing the light beam to scan the plate along a path representing an inoculation track by which the at least one microorganism was inoculated onto the plate. In this way, the data representing the region of the plate is acquired by way of recording the position on the plate at which microorganisms were disposed, such as by an automated or robotic inoculation device, and taking this position to be, or to be proximal to, the position of the target colony once this has been incubated to a sufficient size for analysis by the invention.

The invention is suitable for locating, analysing, characterizing and identifying bacteria in bacterial colonies. Additionally, the method is suitable for use with colonies comprising bacteria, archaea, eukaryotes, protists, rotifers, fungi and yeast cells, and identifying microorganisms comprising any of these.

In accordance with the invention there is also provided a system for identifying microorganisms according to indepndent claim 9, the system comprising inter alia:
a plate upon which colonies of microorganisms may be disposed;
a coherent light source;
a mechanism configured to direct a beam of coherent light emitted by the light source in relation to the plate;
a computer configured to acquire data representing a region on a plate within which at least one colony of microorganisms is disposed, and to control the mechanism so as to expose a target colony of the at least one colony of microorganisms to the beam, in accordance with the data; and
a detector configured to monitor a diffraction pattern resulting from the diffraction of the light by the target colony;
wherein the computer is further configured to generate target colony data in accordance with the monitored diffraction pattern and a reference diffraction pattern.

### Brief Description of the Drawings

Examples of the present invention will now be described, with reference to the accompanying drawings, in which:
Figure 1 is a perspective view showing a first example system for identifying microorganisms according to the invention;
Figure 2 is a flow diagram illustrating a first example method according to the invention;
Figure 3A and Figure 3B are schematic diagrams showing two alternatives of a second example system according to the invention;
Figure 4 is a flow diagram illustrating a second example method according to the invention;
Figure 5 is a flow diagram illustrating a third example method according to the invention;
Figure 6A and Figure 6B are schematic diagrams showing two alternatives of a third example system according to the invention;
Figure 7 is a flow diagram illustrating a fourth example method according to the invention;
Figure 8 is a schematic diagram showing a fourth example system according to the invention;
Figure 9 is a flow diagram illustrating a fifth example method according to the invention;
Figure 10 is a flow diagram illustrating a sixth example method according to the invention;
Figure 11 is a flow diagram illustrating a seventh example method according to the invention;
Figure 12 is a perspective view of a fifth example system according to the invention;
Figure 13 is a flow diagram illustrating an eighth example method according to the invention; and,
Figure 14 is a flow diagram illustrating a ninth example method according to the invention.

### Description of Embodiments

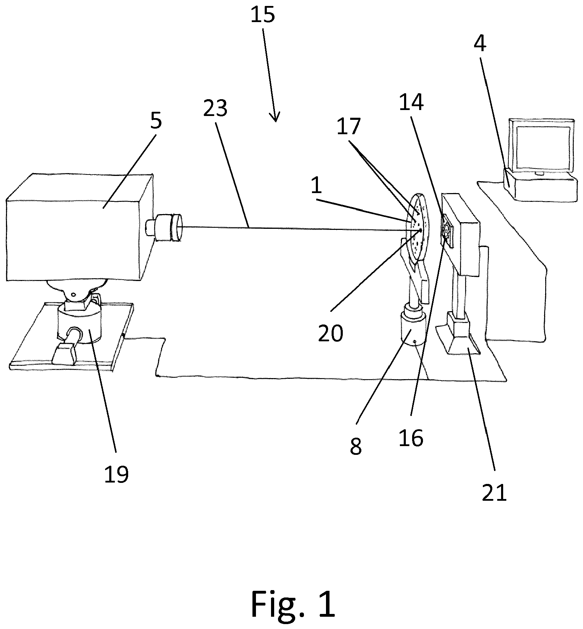

With reference to Figure 1, a system 15 for identifying microorganisms is illustrated by a perspective view showing the main apparatus features of the system suitable for carrying out the method of the invention. The system comprises a laser 5 configured to generate a laser beam, that is a beam of coherent monochromatic light, having a wavelength of the same order as the linear size of the microorganisms or bacteria under study. The laser 5 is arranged to project the beam 23 along a direction corresponding to the optical axis of the system. A transparent plate 1 is positioned on a stand 8 such that the plate is in the optical axis of the system, that is substantially in the path of the beam 23. In the present example, plate 1 comprises an agar plate, which comprises a petri dish containing a growth medium including agar and nutrients, suitable for the culturing of microorganisms. In the presently illustrated example, several colonies 17 of bacteria are disposed on the plate 1. An optical detector 14 comprising a CCD imaging device is mounted on stand 21 and positioned within the optical axis of the system and on the opposite side of the plate 1 to the laser 5. Thus the arrangement is such that the light from the laser 5 may pass through the plate 1 and be received by detector 14.

The laser 5 is mounted on a mechanism 19 which allows the laser to be moved in both up-down and left-right axes with respect to the direction of the beam. That is, the mechanism 19 can move the laser in both of the axes that are orthogonal to the optical axis of the system. Thus the mechanism can cause the laser beam 23 to impinge on any part of the plate 1 by way of moving the light source 5 accordingly. Additionally, the mechanism 19 may move the laser 5 in directions parallel to the optical axis of the system, so as to change the distance between the laser 5 and the plate 1. The laser 5 contains optical elements necessary for modulating the beam 23 so as to narrow or broaden the beam, or focus the beam at a particular point, as may be necessary for optical analysis of bacterial colonies 17.

Additionally, stand 8 may also comprise a mechanism capable of moving the plate in two dimensions within the plate orthogonal to the optical axis of the system. Thus the laser and plate may be moved with respect to one another by moving either or both of the laser and the plate.

Movement of these mechanisms is controlled by computer 4, which is electrically connected to the mechanism 19 as well as stand 8 in the case that stand 8 also comprises a mechanism for moving the plate. The computer is further connected to digital camera device 14, and is configured to receive optical or image data monitored by the detector.

Figure 1 shows a target colony 20 of the plurality of bacterial colonies 17 disposed on the plate, being irradiated by laser beam 23. In the arrangement depicted the mechanism 19 has been controlled by the computer 4 to move the laser 5 such that the beam 23 emitted from the laser impinges upon the target colony 20. The laser device 5 has also been controlled by the computer 4 to confine and focus the beam 23 in order to irradiate target colony 20 specifically without exposing any of the other colonies disposed on the plate 1 to the coherent light 23. The coherent light 23 is diffracted as it passes through the target colony. The pattern of the plurality of bacteria within the colony 20 causes the colony to act as a diffraction element or two-dimensional diffraction grating, and results in the formation of a two-dimensional diffraction pattern 16. The optical parameters of the beam, and the position and size of the detector, are configured and arranged such that the diffraction pattern 16 is received by detector 14, so that image data representative of the pattern 16 may be sent to the computer in order for it to be stored and analysed. Using the system 15, a characteristic diffraction pattern 16 produced by a particular bacterial colony 20 may then be visually compared, by way of computer image analysis techniques, with reference diffraction patterns representative of the equivalent patterns produced by known or predetermined species of bacteria. In this way, the system allows bacteria within individual colonies to be identified in a targeted manner.

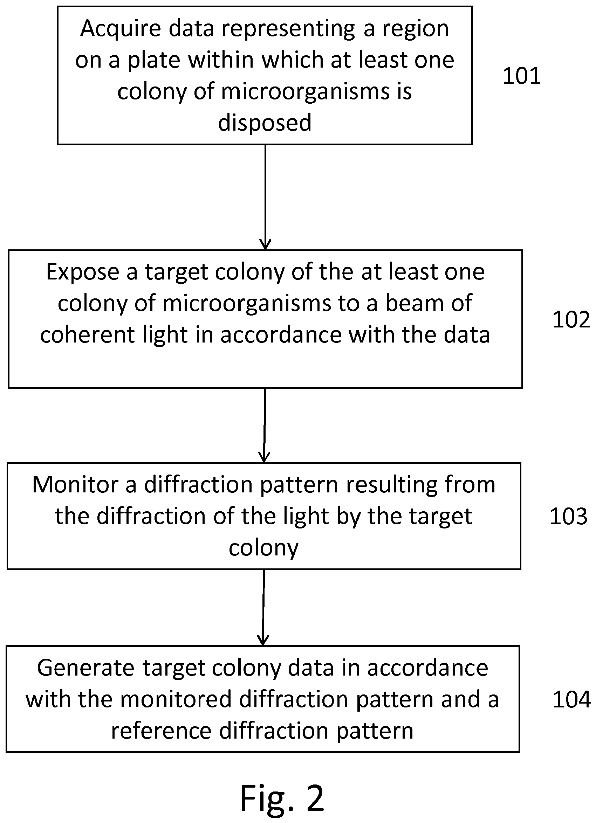

With reference to Figure 1 and Figure 2 a first example method according to the invention, which may be performed using the first example system is now described. The method begins at step 101 in which data is acquired that represents a region on the plate 1 within which at least one colony of microorganisms 17 is disposed. In the presently illustrated example, the data representing the region on the plate comprises optical data representing an image of the region. This data comprises a digital image of a region of the plate 1 that is captured by CCD camera 14. The camera 14 comprises the requisite optical elements for capturing a visual image, or snapshot, of the plate, or a particular region of the plate, such that the bacterial colonies 17, or the spatial distribution of the colonies, is visible within the image. In this way, the image data represents the region.

A target colony 20 of the at least one colony of microorganisms 17 is then exposed to the beam 23 of coherent light, in accordance with the data, at step 102. Focusing the laser beam 23 onto the target colony 20 by using the acquired image data to locate the position of the target colony within the region on the plate, and controlling the mechanism 19 to direct the laser 5 such that the emitted beam coincides with the target colony. The mechanism 19 is controlled by the computer 4. The signal which the computer sends to the mechanism 19 in order to move the laser is generated by the computer based upon the located position of the target colony 20 within the snapshot image acquired by the camera 14 and received by the computer. The snapshot image is analysed using computer algorithms for processing visual information, which distinguish the individual bacterial colonies within the image and acquire the positions of the colonies within the image. The computer is configured with data relating coordinates or positions within the image 2 corresponding physical coordinates or positions on the plate 1, and so the acquired image positions are converted into physical target positions on the plate at which the laser may be directed. Thus the collimated beam of coherent light is caused to irradiate a single selected bacterial colony at a given time, and thereby produce a diffraction image representative of the bacteria present in the single selected colony.

At step 103 the diffraction pattern resulting from the diffraction of the light by the target colony is monitored by the detector 14. In the present example the detector is capable of, and configured for, adjusting its optical and detection configuration to monitor the diffraction pattern in step 103, as well as acquiring the image data at step 101. The monitored diffraction pattern is sent from the detector 14 to the computer 4 via the electrical connection depicted in Figure 1. It will be understood that the wired connections illustrated may readily be substituted for appropriately configured wireless data connections.

At step 104 target colony data is generated by the computer 4 in accordance with the monitored diffraction pattern and a referenced fraction pattern. For the purposes of comparison and identification, a plurality of referenced diffraction patterns is stored on the computer, with each reference pattern corresponding to a known species, type or other taxonomical classification or group of bacteria or other microorganism. The target colony data is generated by pattern analysis software or algorithms executed by the computer which compare the monitored diffraction pattern with any or each of the referenced diffraction patterns in order to establish which of the reference patterns has the greatest degree of visual similarity to the monitored pattern. This is done by way of generating a comparison parameter for each of the reference patterns, and determining the species, type or other classification of the bacteria present in the target colony 20 to be the same as that from which the reference pattern having the greatest comparison parameter was generated.

Once the target colony 20 has been tested, and the species within the colony identified in the target colony data, a second target colony may be selected from the other colonies 17 disposed on the plate. The laser may then be moved with respect to the plate such that the new target colony is irradiated by the beam, and steps 102 to 104 may be repeated for this target colony, as well as any further colonies identified within the region of the plate.

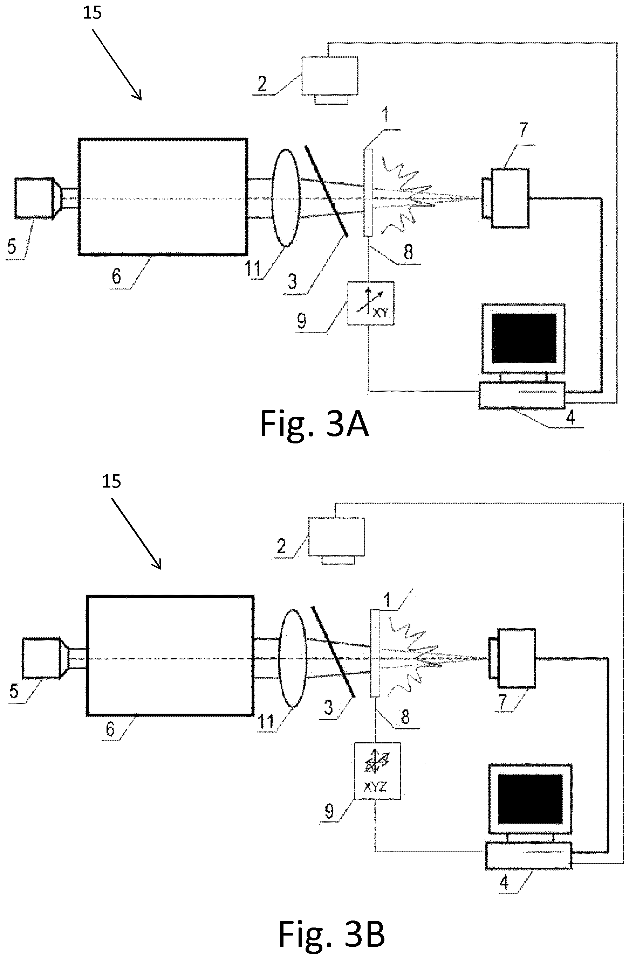

With reference to Figures 3A and 3B, two alternatives of a second example system 15 according to the present invention are illustrated. The measurement system for testing bacteria cultivated on solid media comprises a light source 5 in the form of a laser. An element 6 for modulating a beam of light from the light source 5 is disposed in line with the optical axis of the light source. The modulating element 6 comprises an amplitude filter, a linear polarizer, a beam expander and an iris diaphragm. A converging lens 11 is disposed at the beam output side of the modulating element 6. A transparent plate 1 is disposed further along the optical axis of the system, along with a medium with bacterial colonies. The plate is mounted on a stand 8 comprising a drive 9. The stand 8 comprises a source of radiating light in the form of light-emitting diodes arranged in a ring at the edge of the transparent plate 1. A detector 7 in the form of a camera is disposed, along the optical axis, behind the transparent plate 1. The detector is connected with a computer 4. The computer 4 comprises a system for analyzing diffraction spectra and algorithms for processing visual information, which includes the analysis of colony morphologies. Between the lens 11 and the transparent plate 1, a light splitter 3 is disposed obliquely to the direction of the propagation of the beam of light. The light splitter 3 has the form of a beam splitter cube. In some embodiments, the splitter 3 may comprise a thin glass optical parallel covered by a light splitting film. An additional camera 2 is disposed over the light splitter 3, and is connected with the computer 4. The stand 8 comprises the drive 9 for moving the stand, which allows the stand 8 to be moved, with the transparent plate 1, in directions X and Y perpendicular to the optical axis of the system, as shown in Figure 3A or, additionally, in direction Z parallel to the optical axis of the system, which is indicated in Figure 3B.

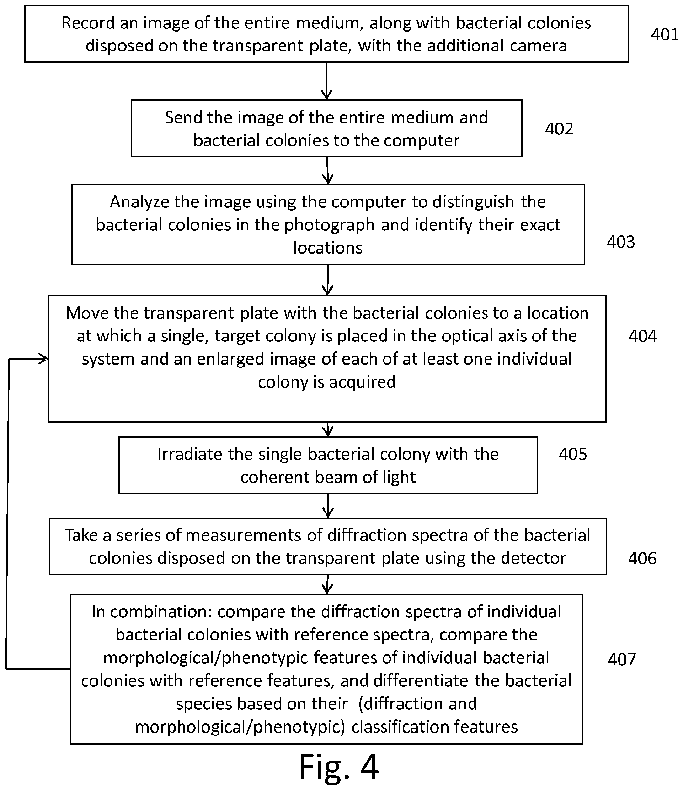

With reference to Figure 4, a second example method according to the invention, which may be carried out in the system as shown in Figure 3A and Figure 3B, is illustrated by a flow diagram. The method begins at step 401 in which an image of the entire medium, along with bacterial colonies disposed on the transparent plate 1, is recorded with the additional camera. The image is recorded from a direction perpendicular to the optical axis of the system, that is a direction parallel to the surface of the transparent plate 1, with the additional camera 2 by recording the beam reflected from the light splitter 3. The light splitter 3 is situated, in the presently described example, at an angle of 45 degrees to the optical axis of the system, and the additional camera 2 is situated perpendicularly to the optical axis of the system. It is possible to take a photograph by way of illumination of the subject by radiation emitted from the source within the stand 8. At step 402 the image of the entire medium and bacterial colonies is sent to the computer 4. It is then analyzed by the computer 4, at step 403, using computer algorithms for processing visual information. The bacterial colonies in the photograph are distinguished and their exact locations are identified. At step 404 the transparent plate 1 with the bacterial colonies is then moved to a location at which a single, target colony is placed in the optical axis of the system. The plate is moved by way of changing the location of the stand 8 on which the plate is mounted, using the drive 9 which controls the movement of the stand 8 in directions X and Y perpendicular to the optical axis of the system, as shown in Figure 3A, or in three directions: along the optical axis of the system and in directions X and Y perpendicular to the optical axis of the system, as shown in Figure 3B. At step 405, the single bacterial colony is then irradiated with the coherent beam of light that projects from the light source 5 and passes through the element 6, which modulates the beam. At step 406, using the detector 7, which has the form of a camera, a series of measurements of diffraction spectra of the bacterial colonies disposed on the transparent plate 1 are taken. At step 407 the diffraction spectra of individual bacterial colonies are compared with reference spectra and, based on their classification features, the bacterial species is differentiated. The transparent plate 1 with the bacterial colonies is then moved, using the drive 9 of the stand 8 on which the plate is mounted, to a location at which another single colony is positioned in the optical axis of the system, as in step 404. That bacterial colony is then irradiated with the coherent beam of light which projects from the light source 5 and passes through the modulating element, as in step 405. The steps up to 407 are repeated, until each colony has been irradiated with the convergent beam of coherent light generated by the source 5 and modulated by the modulating element 6, which comprises optical elements that change the diameter, intensity and wavefront of the beam.

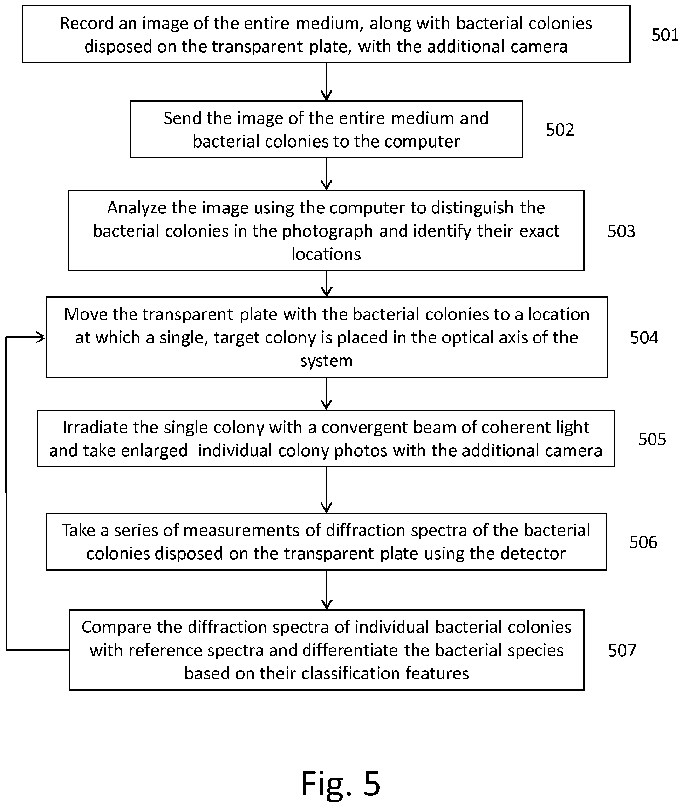

With reference to Figure 5, the flow diagram shows a third example method according to the invention, which has several steps (501 to 504) in common with the second example method described above. At step 504, after distinguishing and identifying the exact location on the transparent plate 1 of the bacterial colonies recorded, the transparent plate 1 is moved, using the drive 9 of the stand 8 in which the plate is mounted, and at step 505 a single colony is irradiated with a convergent beam of coherent light generated by the light source 5 and, with the additional camera 2, enlarged photos of individual colonies are taken. Then, at step 506 the diffraction spectra of that colony are detected and the spectra are compared with reference spectra. In the present example, the additional camera 2 comprises two lenses. One of the lenses has a variable focal length, and the camera 2 therefore takes the enlarged photograph of the single bacterial colony and the images of the entire medium of the transparent plate 1 along with the bacterial colonies. The algorithms for processing visual information of the computer 4 allow the morphology of the bacterial colonies to be analyzed.

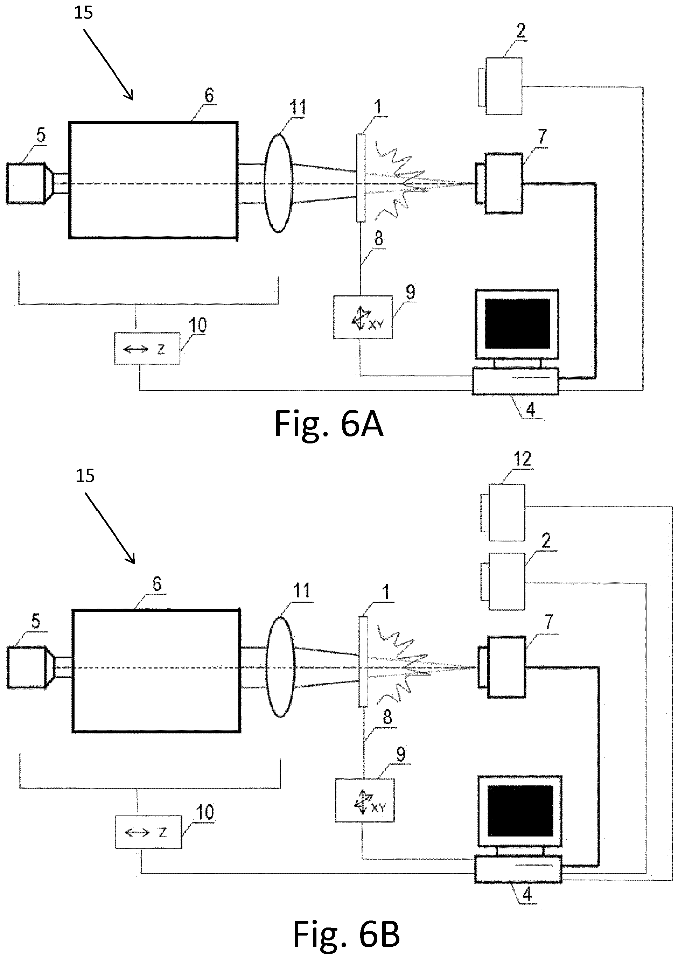

Figure 6A and Figure 6B show two alternatives of a third example system 15 according to the invention. The system comprises light source 5, in the form of a laser, and an element 6 for modulating the beam of light projecting from the light source 5, disposed along the optical axis of the source. The element comprises an amplitude filter, a linear polarizer, a beam extender and an iris diaphragm and, at the outlet of the beam of light from the modulating element 6, a converging lens 11 is disposed. Further along the optical axis of the system, transparent plate 1 with bacterial colonies is mounted on stand 8. The stand 8 comprises a source of radiating light comprising light-emitting diodes disposed in the form of a ring at the edge of the transparent plate 1. The stand 8 also has a drive 9 for moving the stand in directions X and Y perpendicular to the optical axis of the system. Behind the transparent plate, with respect to the source of the beam, and positioned along the optical axis, detector 7, in the form of a camera, is disposed and is connected with computer 4. The computer 4 comprises a system for analyzing the diffraction spectra and algorithms for processing visual information, also including analysis of colony morphologies. Additional camera 2 is disposed beside detector 7 and is also connected with the computer 4. The additional camera 2 is disposed in parallel to the camera 7, along the optical axis of the system. The coherent light source 5, along with the element 6 for modulating the beam and the lens 11, comprise the second drive 10 which allows the entire unit composed of the elements mentioned above, to be moved as a whole, in direction Z parallel to the optical axis of the system.

In the second alternative of the third example system 15, as shown in Figure 6B, the system depicted in Figure 6A further comprises an additional detector 12 for recording additional signals. The additional detector 12 and the additional camera 2 are situated in parallel to the camera 7 disposed along the optical axis of the system. The light source 5, the modulating element 6 and the transforming lens 11 are integrated with the second drive 10, which automatically moves these elements in direction Z parallel to the optical axis of the system. The additional detector 12 may have the form of a camera and be used to record enlarged images of individual bacterial colonies.

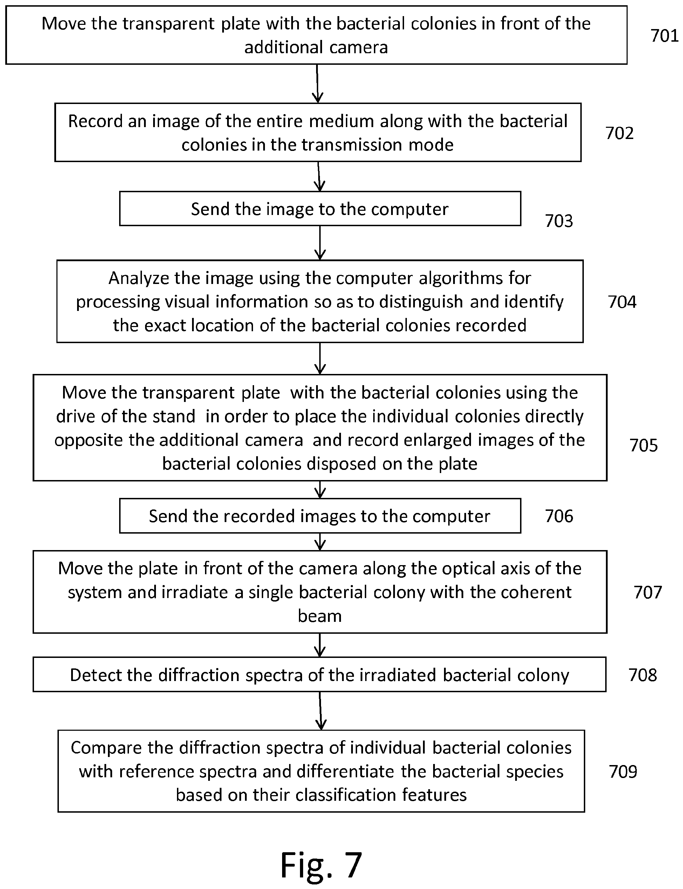

A fourth example method according to the invention, relating to the system as shown in Figure 6A and Figure 6B is shown by the flow diagram of Figure 7. At step 701 the transparent plate 1 with the bacterial colonies is moved in front of the additional camera 2, and at step 702 an image of the entire medium along with the bacterial colonies in the transmission mode is recorded. That image is sent to the computer 4 at step 703 and is analyzed using the computer algorithms for processing visual information so as to distinguish and identify the exact location of the bacterial colonies recorded at step 704. Then, at step 705, the transparent plate 1 with the bacterial colonies, mounted on the stand 8, is moved using the drive 9 of the stand 8, in order to place the individual colonies directly opposite the additional camera 2 and enlarged images of the bacterial colonies disposed on the plate, are recorded. At step 706 the recorded images are sent to the computer 4. At step 707 the transparent plate 1 with the bacterial colonies is moved, again using the drive 9 to move the stand 8, in front of the camera 7 disposed along the optical axis of the system, and a single bacterial colony is irradiated with the coherent beam of light. Then, the diffraction spectra of the irradiated bacterial colony are detected at step 708, the spectra are recorded and are compared with reference spectra at step 709. In this system, the stand 8 comprises a drive 9 allowing for moving it automatically in directions X and Y perpendicular to the optical axis of the system. Moreover, the light source 5, the modulating element 6 and the transforming lens 11 are integrated with the second drive 10 which automatically moves these elements in direction Z parallel to the optical axis of the system. The distances between these elements are set, and the whole assembly moves in order to adjust the diameter of the radiating beam to the diameter of the colony.

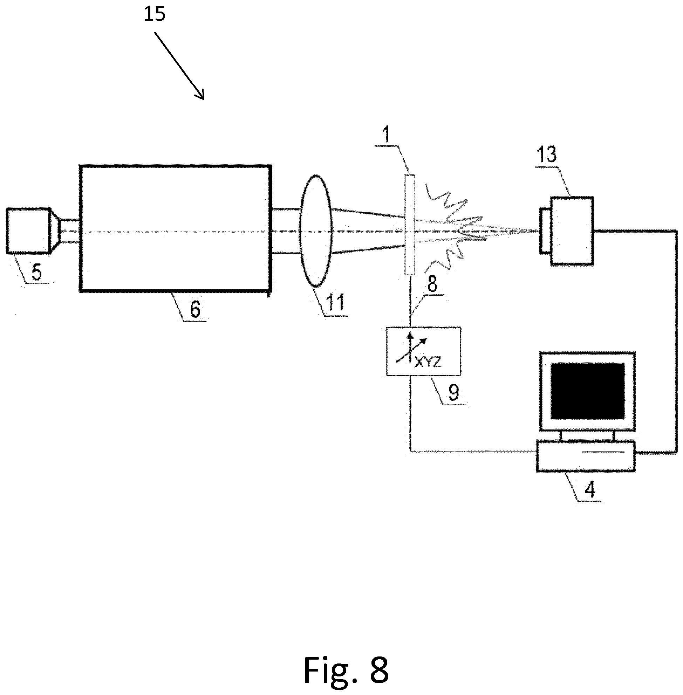

As shown in Figure 8, a fourth example system 15 according to the invention is shown. The system comprises light source 5 in the form of a laser, modulating element 6 disposed along the optical axis of the beam of light projecting from the source 5. The element comprises an amplitude filter, a linear polarizer, a beam expander and an iris diaphragm. A converging lens 11 is disposed at the outlet of the beam of light from the modulating element 6. Transparent plate 1 with bacterial colonies is disposed further along the optical axis of the system, mounted on stand 8 comprising drive 9. Detector 13 in the form of a camera is disposed behind the transparent plate 1 along the optical axis, and is connected with computer 4. The computer 4 comprises a system for analyzing diffraction spectra and algorithms for processing visual information which includes the analysis of colony morphologies. The detector 13 is a camera comprising two lenses, and one of these lenses has a variable focal length, which allows the recording of the photograph of the entire transparent plate 1 with the bacterial colonies, the enlarged photos of individual colonies, and diffraction spectra. In present example, the image of the entire medium along with the bacterial colonies is recorded from a direction perpendicular to the surface of the transparent plate 1. The drive 9 allows for the moving of the stand 8 in the direction parallel to the optical axis of the system and in directions X and Y perpendicular to the optical axis of the system.

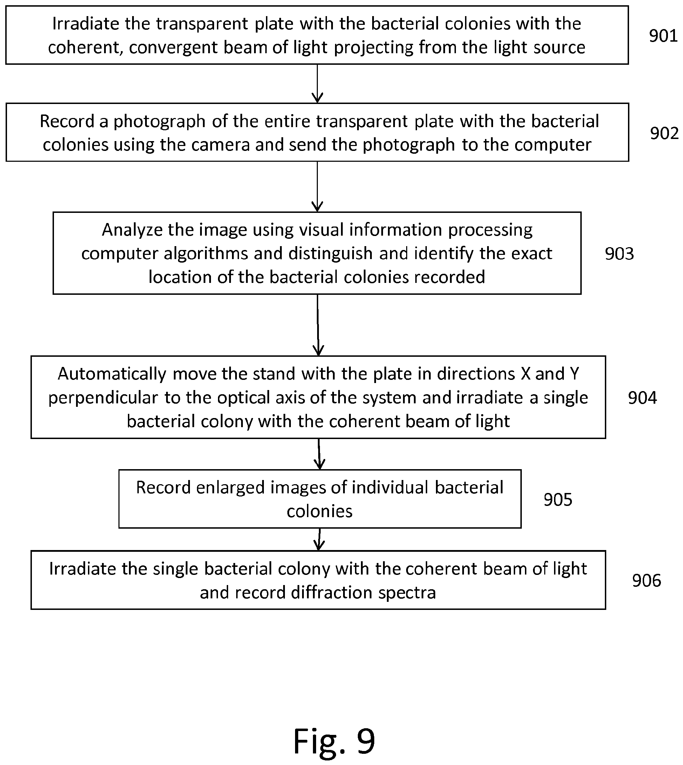

Steps of a fourth example method according to the invention are shown by the flow diagram in Figure 9. At step 901 the transparent plate 1 with the bacterial colonies is irradiated with the coherent, convergent beam of light projecting from the light source 5, and then, at step 902 a photograph of the entire transparent plate 1 with the bacterial colonies is recorded using the camera 13 and is sent to the computer 4. Then, at step 903 the image is analyzed using the computer algorithms for processing visual information and the exact location of the bacterial colonies recorded is distinguished and identified. Then, at step 904 the stand 8 is moved automatically with the transparent plate 1 with the bacterial colonies in directions X and Y perpendicular to the optical axis of the system, and a single bacterial colony is irradiated with the coherent beam of light. At step 905 enlarged images of individual bacterial colonies are recorded. Then, at step 906 the single bacterial colony is irradiated with the coherent beam of light and diffraction spectra are recorded. The stand 8 is moved automatically in directions X and Y perpendicular to the optical axis of the system using the drive 9.

The rapid, efficient, non-destructive and contactless testing of bacterial colonies may be accomplished by the invention by way of the image of the transparent plate 1 with the bacterial colonies being recorded from a direction perpendicular to the optical axis of the system of the location of the transparent plate by recording the beam reflected from the light splitter, with the image being sent to the computer and analyzed using the computer algorithms for processing visual information and the exact location of the recorded bacterial colonies being distinguished and identified. The data representing the distribution of the individual bacterial colonies on the transparent plate 1 allows for the moving of the transparent plate 1 in such a way that the single target bacterial colony tested may be irradiated with the coherent beam of light, and then the diffraction spectra of the bacterial colony irradiated may be measured and compared with reference spectra.

The provision of the stand 8 with the drive 9 allows for the automatic and rapid change in the location of the stand 8 while facilitating rapid and precise measurement of the diffraction spectra of the bacteria cultivated. The additional recording of the photograph of the entire transparent plate 1 with bacterial colonies provides information about the colony count, which in turn, in conjunction with the recorded Fresnel diffraction spectra of the tested bacterial colonies, allow for the determination of a specific bacterial species. The visual match correlation or quality or a degree of confidence in the characterization or identification achieved with the target colony data may be quantified as a percentage, for instance.

The provision of the optical system (a light source 5, a modulating element 6 and the transforming lens 11) with the second drive 10 allows for the changing of the location of the optical system in relation to the transparent plate 1 with the bacterial colonies.

Adjustment of the radiating beam diameter to the diameter of the bacterial colony tested is obtained by the provision of the unit comprising the coherent light source 5 along with the modulating element 6 and the lens 11 with the second drive 10 in the direction parallel to the optical axis of the system.

The provision of the stand 8 with the source of radiating light allows for the rapid acquisition of photographs of the entire transparent plate 1 with the bacterial colonies.

Recording the enlarged images of individual colonies is possible with the camera having two lenses or the camera having a focal length that allows for determining the phenotypic characters of the bacterial colony tested. The determination of these additional phenotypic characters along with the measurement of the Fresnel diffraction spectra of the bacterial colony tested allows a higher number of characters differentiating the bacterial species and/or strain to be distinguished.

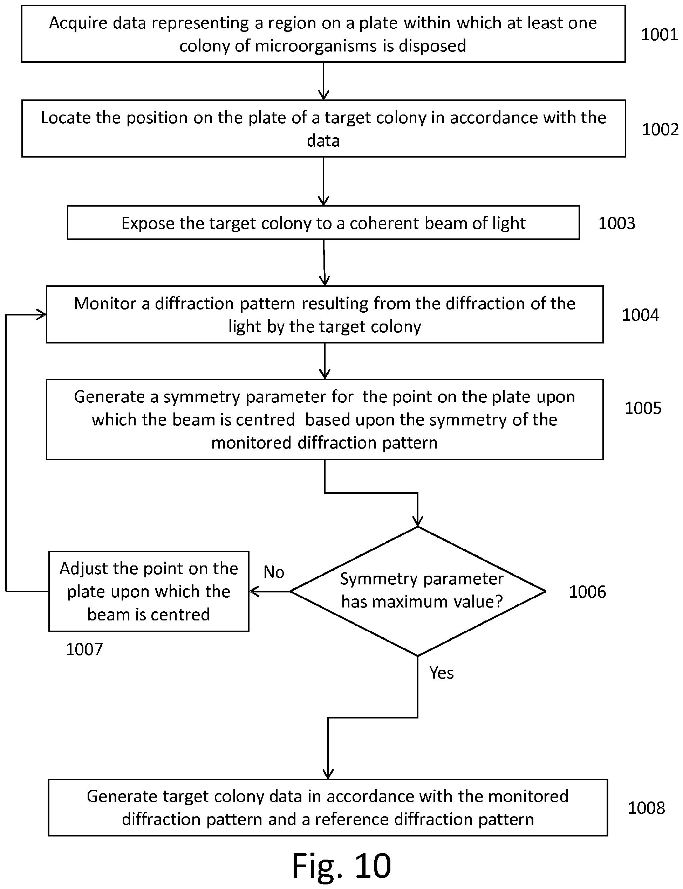

Figure 10 shows the steps of a sixth example method according to the invention wherein the diffraction analysis is improved by a way of precisely centering the laser beam upon an optimal point of the target colony. The method begins at step 1001, wherein data representing a region on a plate within which at least one colony of microorganisms is disposed is acquired. This may be performed via any techniques similar to those described in the previous examples. At step 1002 the position on the plate of a target colony is located in accordance with the data, likewise in a manner corresponding to the previously described examples. At step 1003 the laser is directed to expose the target colony to a coherent beam of light, based upon the located position from the previous method step. In the present example, the target colony data is not necessarily immediately generated based upon the diffraction pattern monitored at this step. At step 1004 the diffraction pattern resulting from the diffraction of the light by the target colony is monitored by a detector. However, rather than immediately generating a comparison parameter or target colony data, a symmetry parameter is generated at step 1005. The generated parameter is for the point on the plate upon which the beam is centered, and is based upon the symmetry of the monitored diffraction pattern. This is achieved by image analysis techniques executed by the computer. The computer may be configured to assess and quantify the symmetry, homogeneity, size, contrast ratio, or sharpness of the monitored diffraction pattern, for example, in generating the symmetry parameter. The parameter is generated in such a way that it indicates whether the alignment between the laser and the target colony is optimal for achieving a symmetrical, high quality diffraction pattern. It may be performed using energy centroid calculation, wherein a center of the energy of the diffraction image or a portion of an image is determined using computer algorithms. If the energy center is not aligned with the center of the image, the diffraction is not indicated to be homogeneous, and is likewise not ultimately symmetrical.

Reasons for non-optimal symmetry parameters indicating misaligned laser beams may include sub-optimally positioned lasers and a cluster colony, that is a group of bacteria colonies growing together. At step 1006 the computer assesses whether the symmetry parameter has a maximum value or exceeds a predetermined threshold, which indicates that the centroid has been found. If the parameter does not fulfill the criteria, the point on the plate upon which the beam is centered is adjusted at step 1007. This is achieved by moving the laser by degrees corresponding to positions on the plate of the order of fractions of the linear size of the target colony. Steps 1004 to 1006 are then repeated with the laser having been repositioned by a fractional amount in order to seek the optical centroid of the colony at a slightly different point within the colony. By repeating these steps and moving the point at which the central axis of the laser coincides with the target colony until the symmetry parameter is maximized, the optical centroid is located by way of an iterative seeking process. Once the symmetry parameter has reached a maximum value, the diffraction pattern generated by centering the optical axis of the beam is monitored, and target colony data is generated in accordance with the monitored diffraction pattern and a reference diffraction pattern at step 1008. Thus the monitored pattern for a particular target colony which is used to identify that colony is generated by centering the laser upon the optimal optical centroid rather than simply selecting to direct the beam towards the geometric centroid of the colony, which may produce a less symmetrical, less representative and therefore less useful diffraction image.

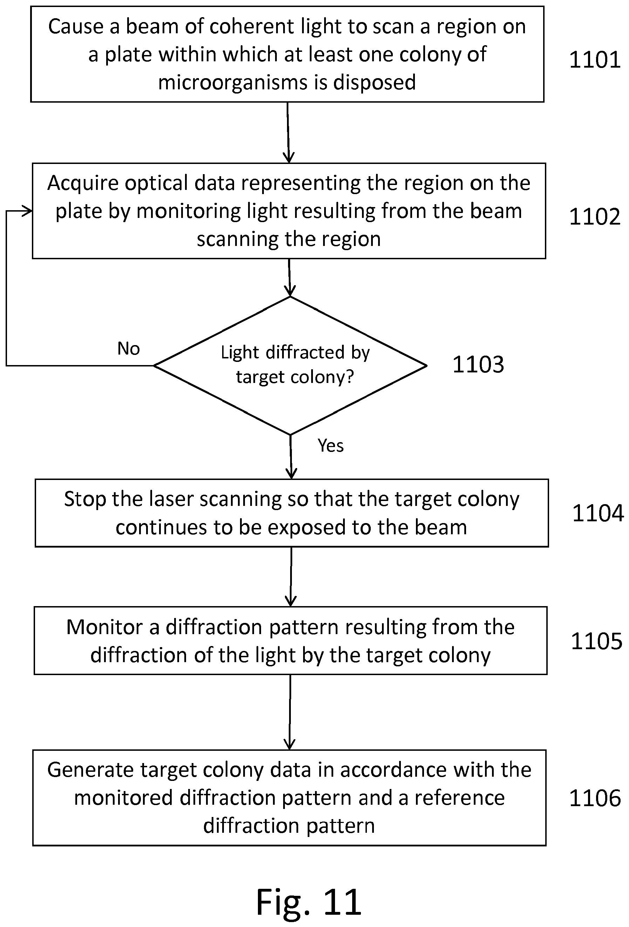

With reference to Figure 11 a seventh example method according to the invention is illustrated by a flow diagram. This example method involves acquiring data which is used to locate and direct the laser towards a target colony is acquired by scanning the laser across the plate rather than using a camera to acquire an image of the plate using a single exposure.

At step 1101 a beam of coherent light is caused to scan a region on a plate within which at least one colony of microorganisms is disposed. Thus, instead of using a camera to acquire a snapshot of the plate in order to locate the bacterial colonies thereupon, the laser begins scanning across the plate. This may be achieved by moving one or both of the laser and plate by way of an automated mechanism, such as in the previously described examples, and may be performed in a regular scanning pattern such as raster scanning, or in a random pattern. The data representing the region on the plate is thus acquired by monitoring the light resulting from the beam scanning across the region of the plate. This optical data is acquired by a detector such as that illustrated in the previous figures at 14. In the current example, since a camera snapshot is not required for locating the target colony, the requisite optical detector may be simpler than that required for methods including both snapshots and diffraction pattern monitoring. While the laser is scanning and the light passing through the plate is being monitored by the detector, the detector sends a signal representing the monitored pattern to a computer, which assesses whether the received image corresponds to a diffraction pattern. This decision, indicated at 1103, is made based upon processing of the monitored image by the computer and comprises comparing the optical data received with characteristic visual properties of colony diffraction patterns to identify whether the laser light passing through the plate at a particular point is being diffracted by a colony or not. The scanning continues, that is the method returns to step 1102, until it is established that the monitored pattern corresponds to the pattern expected when the laser is diffracted by a target colony, or a colony of a particular type. The method then proceeds to step 1104, and the laser stops scanning at the point corresponding to the detected diffraction pattern, so that the target colony which caused the diffraction continues to be exposed to the beam.

The method then continues, at steps 1105 and 1106, in monitoring and generating target data based upon the diffraction pattern resulting from the target colony affecting the beam.

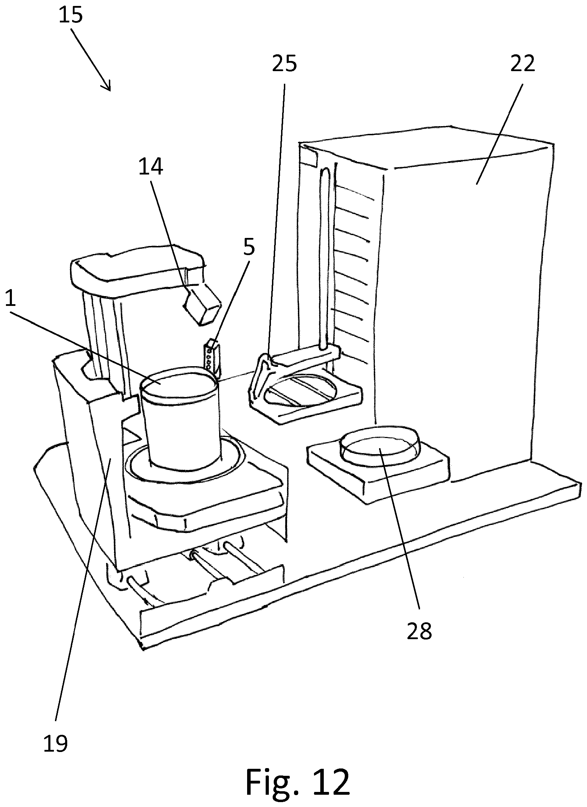

With reference to Figure 12 a fifth example system for identifying bacteria according to the invention is illustrated by a perspective view. The present example system comprises the same main features as those illustrated in the previous figures, and further includes apparatus for the automated inoculation and incubation of colonies upon the plate.

In line with previous examples, the system 15 includes a transparent agar plate 1, positioned such that it, as well as any cultured bacterial colonies, may be illuminated by illuminator 5. This illuminator is configured for providing illumination in order for the initial snapshot of the plate to be taken, as well as for causing a beam of coherent light to irradiate the disposed colonies. An optical detector 14 is positioned within the integrated system such that it is above the horizontally aligned plate and has a field of view directed down upon the plate. The mechanism 19 comprises robotic actuators which are configured to move the plate, laser, and optionally the detector 14, with respect to one another. Thus the system is capable of acquiring data so as to locate a target colony upon a plate and positioning the monitoring and illumination apparatus so as to target that particular colony once located. The system further comprises a robotic manipulator 25. This is positioned and configured for depositing samples of bacteria from input area 28 onto a growth medium within a transparent plate such as plate 1. This also comprises a delivery mechanism for delivering plates upon which colonies have been cultured from incubation tower 22 to the analysis area proximal to light source 5 and detector 14. The manipulator 25 may inoculate bacterial samples onto a plate, so that these plates may be moved via an automated mechanism into the incubation tower 22, wherein the plates may be incubated so as to encourage the development of bacterial colonies from the initially deposited samples. The inoculation of bacteria onto plates, the incubation of the plates, the diffraction analysis and generation of target colony data and identification, and all movements within the system required for these steps may be performed automatically by the robotic actuators and manipulators of the integrated system 15. The entire process is controlled by a computer (not shown) which is also integral to the system, but may alternatively be separate from the system and issue controlling commands and receive feedback via data connections.

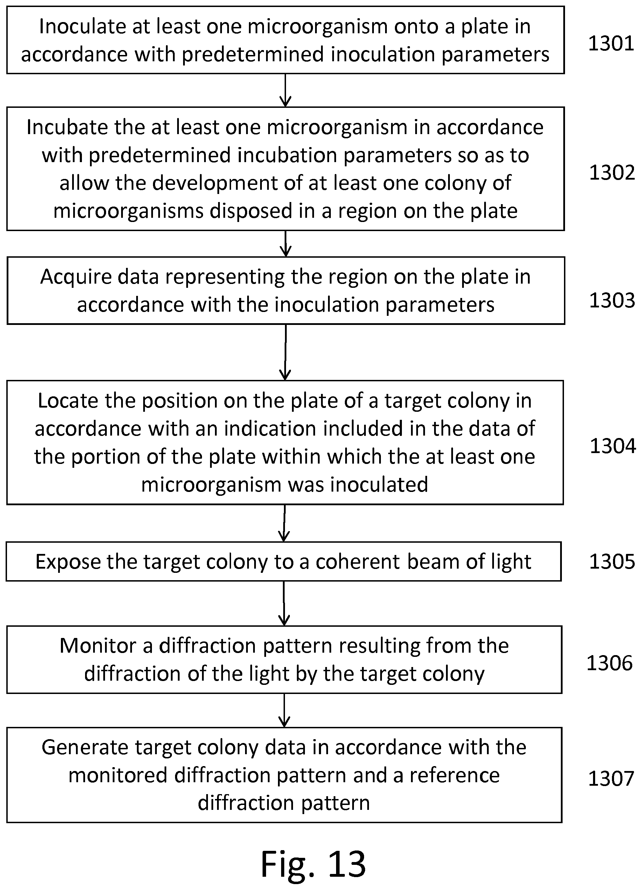

Figure 13 is a flow diagram illustrating an eighth example method according to the invention which may be performed by the integrated inoculation, incubation and analysis system depicted in Figure 12. The method begins at step 1301 wherein at least one microorganism is inoculated onto a plate in accordance with predetermined inoculation parameters. These inoculation parameters may comprise programmed instructions for controlling the robotic inoculator so as to perform this step in a consistent, repeatable manner. In the present example the step comprises the manipulator 25 taking a bacterial sample from input area 28 and disposing it upon an agar plate. The plate is then moved, automatically, by the mechanisms comprised by the system, into the incubation tower 22, and the at least one microorganism is incubated, at step 1302, in accordance with predetermined incubation parameters so as to allow the development of at least one colony of microorganisms disposed in a region on the plate. It will be understood that the programmed parameters may include computer instructions for maintaining certain temperatures within the tower, or incubating the plates for certain periods of time.

Once the colonies have been incubated for a length of time sufficient for them to have reached a size large enough that a diffraction pattern may be produced from them, the delivery mechanism of the system removes the plate to be examined from the tower 22 and mounts it on mechanism 19.

The system may then acquire data representing the region on the plate and allowing a target colony to be located via techniques similar to those described already. In the present example, however, the data representing the region on the plate is acquired in accordance with the inoculation parameters. This process, at step 1303, comprises obtaining, from the stored incubation parameters, an indication of the point or portion of the plate upon which the inoculating device was programmed to deposit the initial bacterial example. Using this information, at step 1304 the position on the plate of a target colony is located in accordance with an indication included in the data of the portion of the plate within which the at least one microorganism was inoculated. Using this technique, the automated process executed by the illustrated system may be carried out more quickly, since no separate scanning or photograph capturing step is required. The positions of the colonies on the plate may be known before the analysis begins.

The diffraction analysis steps indicated at steps 1305, 1306 and 1307 are then performed similarly to the corresponding steps in the previously described examples.

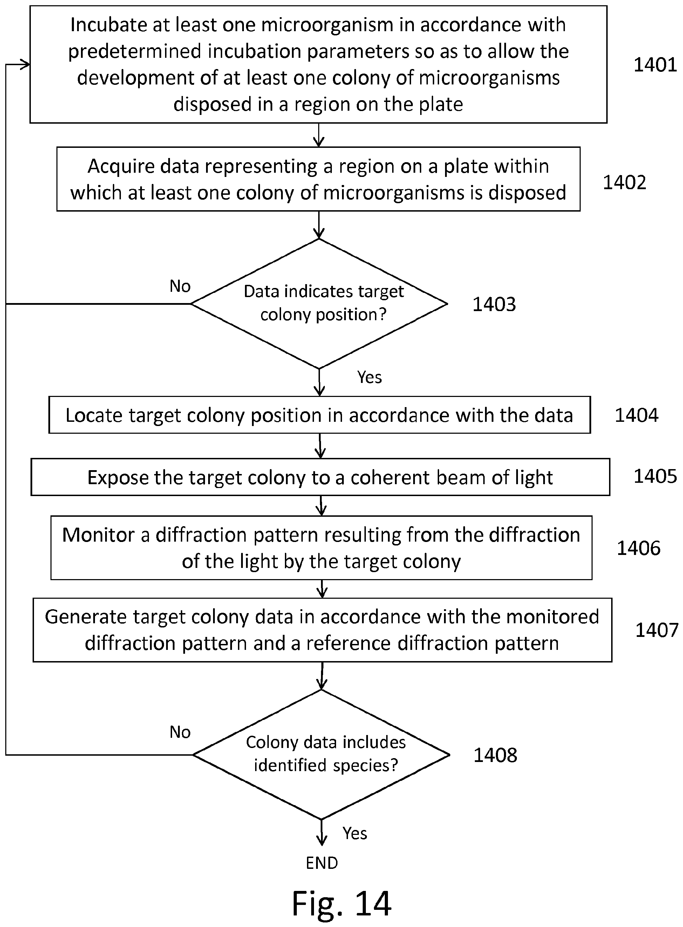

With reference to Figure 14, the flow diagram illustrates a ninth example method according to the invention, wherein a time-course recognition technique is employed. At step 1401 at least one microorganism is incubated in accordance with predetermined incubation parameters so as to allow the development of at least one colony of microorganisms disposed in a region on a plate. This may be performed similarly to the incubation step in the previously described example. However, in the present example, the plate is periodically removed from the incubation tower 20 and analysed by methods in line with those described above, in order to establish whether the development of the colonies has thus far been sufficient for the colonies to be located and identified.

The plate is removed from the incubator and data is acquired representing a region, the plate within which at least one colony of microorganisms is disposed at step 1402. This data acquisition step may be performed by an optical means such as the laser scanning or photograph capturing described in the above examples. If the acquired data does not indicate the position of a target colony, the plate is returned to the incubator, and steps 1401 to 1403 are repeated. It may be determined, at step 1403, that the data thus far lacks any indication of the target colony position because insufficient incubation time has elapsed and the colonies are of a size that is too small to be detected by visual or optical means. If necessary, this process is repeated until a target colony position is indicated by the data. Once this has occurred, the target colony position is located in accordance with the data at step 1404.

With the target colony located, the plate 1 is positioned such that the colony is exposed to a beam of coherent light at step 1405. The diffraction pattern resulting from the diffraction of the coherent light by the target colony is monitored at step 1406, and target colony data is generated at step 1407 in accordance with the monitored diffraction pattern and a reference diffraction pattern, by a method similar to those described previously.

At step 1408 the computer 4 makes a determination of whether the colony data includes an identified species, that is whether the species is identifiable from the data by the computer with reference to the predetermined reference patterns. If this is not the case, the plate is removed from the analysis area and returned to the incubation tower so that further incubation can be performed, returning the method to step 1401. The steps from 1401 are then repeated until the computer can establish at step 1408 that the colony data includes an identified species of bacteria. When the species has been identified, the method ends.

The present example allows more efficient location and identification of bacteria by way of repeating the indicated steps so as to identify the species present as soon as the colonies have developed sufficiently to be identified by visual and diffraction analysis. Using this method the growth of bacteria colonies may be monitored such that data is acquired on an hourly or two hourly basis, for example, until the species is recognizable.

Additionally, time-course diffraction images may be used as additional information for identifying bacteria. This technique involves repeating the analysis steps as indicated in Figure 14 in order to monitor how the diffraction image of a particular colony changes with time. More diffraction data may thus be acquired, in addition to data indicating the growth and development properties of the colonies as indicated by the optical data obtained.

## Claims

1. A method of identifying microorganisms comprising any of bacteria, archaea, eukaryotes, protists, rotifers, fungi and yeast cells, the method comprising automatically performing the steps of:
- inoculating at least one microorganism onto a plate; and incubating the at least one microorganism so as to allow the development of at least one colony of microorganisms disposed in a region on the plate, wherein each of the inoculating and incubating is performed by an automated system in accordance with predetermined inoculation parameters and incubation parameters, respectively and further comprising:
- acquiring data representing the region on the plate comprising a culture medium, and the at least one colony of microorganisms is disposed on the medium;
- locating the position on the plate of a target colony of the at least one colony of microorganisms, in accordance with the data;
- acquiring at different times data comprising an enlarged image of each of at least one individual colony being target colony;
- exposing the target colony to a modulated coherent light beam, in accordance with the data;
- monitoring at different times a diffraction pattern resulting from the diffraction of the light by the target colony; and
- generating target colony data at different times indicating a species present in the target colony in accordance with monitored diffraction pattern and a reference diffraction pattern
wherein the target colony data is generated by comparing the monitored diffraction pattern with the reference diffraction pattern comprising generating a comparison parameter based upon a comparison of the monitored diffraction pattern with each of a plurality of reference diffraction patterns, each reference diffraction pattern corresponding to a predetermined species; and identifying the target species present in the target colony as the predetermined species for which the generated comparison parameter has the greatest value and and wherein the modulated coherent light beam is configured to scan the plate along a path representing an inoculation track by which the at least one microorganism was inoculated onto the plate, wherein the modulated coherent light beam is a light generated by a source (5) of coherent light modulated by a modulating element (6), which comprises optical elements that change the diameter, intensity and wavefront of the beam of the coherent light from source (5).

2. A method according to claim 1, wherein a part of the region on the plate upon which the beam of coherent light impinges is adjusted by adjusting either or both of: the direction of the modulated coherent beam; and the position of the plate, what is achieved by directing the modulated coherent light beam towards optical centroid defined as a point within the target colony corresponding to the greatest value of a symmetry parameter related with the symmetry, contrast or homogeneity of the monitored diffraction pattern, such that the modulated coherent light beam coincides with the position on the plate of the target colony, and correcting the relative positions until the modulated coherent light beam coincides with a point corresponding to a maximum value of the symmetry parameter.

3. A method according to claim 2 or 1, wherein acquiring the data comprises acquiring an image of the region on the plate by exposing the region on the plate to radiation and acquiring an image of the region and the at least one colony of microorganisms, wherein the image is recorded from a direction perpendicular to the surface region of the plate within which the at least one colony is disposed or the image is recorded from the direction parallel to the surface of the plate by recording the modulated coherent light beam reflected from a light splitter (3) and locating the position on the plate (1) of the target colony is achieved by analyzing the acquired image using a computer (4) configured to process image data so as to identify and locate the position of a colony of microorganisms within the image.

4. A method according to any of the preceding claims, wherein acquiring the data comprises causing the modulated coherent light beam to scan the region on the plate (1) and locating the position on the plate of the target colony is performed by monitoring light resulting from the modulated coherent light beam scanning the region on the plate (4) and detecting light diffracted by the target colony.

5. A method according to any of the preceding claims, wherein prior to the monitoring of the diffraction pattern of the target colony:
the at least one colony of microorganisms disposed within the region on the plate is irradiated;
an image of the entire plate and the at least one colony of microorganisms is recorded, sent to a computer, analyzed using computer algorithms for processing visual information, and the location of the at least one colony of microorganisms recorded is distinguished, and then:
the plate and the at least one colony of microorganisms is moved relative to an optical system comprising a source of the coherent light beam; and
a single colony of microorganisms of the at least one colony of microorganisms is irradiated with the modulated coherent light beam.

6. A method according to claim 5, wherein the algorithms for processing visual information comprise analysis of colony morphologies extending the target colony data related to or indicating the physical or apparent shapes and geometric properties of microbial colonies are applied to the enlarged image;
and used in combination with diffraction analysis data in order to identify or characterize microorganisms within the target colony;
wherein the target colony data is generated by comparing the monitored diffraction pattern with the reference diffraction pattern and and preferably
the step of acquiring the data is repeated at least until the acquired data indicates the position on the plate of the target colony and exposing the target colony to the beam, monitoring the diffraction pattern, and generating target colony data are repeated at least until the target colony data includes an identified species present in the target colony and more preferably
the steps are repeated according to a predetermined time interval and over a sufficiently long period of time for a colony of microorganisms to grow on the plate, and wherein the data indicating the position on the plate of the target colony and the target colony data includes an identified species present in the target colony are defined by the target colony having grown to such a size that it may be visually located in accordance with the data and the diffraction pattern contains sufficient data to identify the species with reference to a reference diffraction pattern.

7. A method according to claim 6, wherein the target colony data is generated in accordance with two or more monitored diffraction patterns wherein the two or more patterns are monitored at different times and are representative of the diffractive properties of the target colony and their changes at the different times.
and inoculation and incubation parameters are configured such that, each time the method is performed, the physical processes by way of which the colonies of microorganisms are inoculated and incubated are performed identically.

8. A method according to any of claims 7, wherein the data representing the region on the plate is acquired in accordance with the inoculation parameters, such that the data includes an indication of the portion of the plate within which the at least one microorganism was disposed; and the position on the plate of the target colony is located in accordance with the indicated portion of the plate or the light beam is scanning the plate along a path representing an inoculation track by which the at least one microorganism was inoculated onto the plate.

9. A system for identifying microorganisms, the system comprising:
- a plate (1) upon which colonies of microorganisms (17) may be disposed
- an inoculator device for inoculating at least one microorganism onto the plate along an inoculation track;
- an incubator (22) for incubating the at least one microorganism so as to allow the development of at the least one colony of microorganisms disposed on the plate;
- a coherent light source (5) emitting a coherent light beam (23);
- a mechanism (19) configured to direct a beam of coherent light emitted by the light source in relation to the plate; a modulating element (6) which comprise elements configured to change the diameter, intensity and wavefront of the coherent light beam (23) to generate a modulated coherent light beam;
- a computer (4) configured to acquire data representing a region on a plate within which at least one colony of microorganisms is disposed and indicating the position on the plate of the target colony, locate the position on the plate of the target colony of the at least one colony of microorganisms in accordance with the data, and control the mechanism (19) so as to expose the target colony to the beam and
- a detector (14) configured to monitor at different times a diffraction pattern (16) resulting from the diffraction of the light by the target colony;
wherein the computer is further configured to generate target colony data, comprising indication of a species present in the target colony in accordance with the monitored diffraction pattern and a reference diffraction pattern;
and wherein the detector (14) is further configured to acquire image data representing an image of the region in response to radiation incident upon the detector, and wherein the data representing the region on the plate comprises the image data; and wherein the computer is configured to locate the position on the plate of the target colony of the at least one colony of microorganisms in accordance with the image data, and generate a comparison parameter based upon a comparison of the monitored diffraction pattern with each of a plurality of reference diffraction patterns, each reference diffraction pattern corresponding to a predetermined species; and to identify the target species present in the target colony as the predetermined species for which the generated comparison parameter has the greatest value *and wherein* the system is configured to scan *the modulated* coherent *light beam along a path representing* the *inoculation track by which the at least one microorganism was inoculated onto the plate* .

10. A system according to claim 9 , wherein the mechanism (19), comprising robotic actuators configured to move plate (1) and the coherent light source (5), is configured to expose the target colony (20) to the beam of coherent light I (23) by directing the light source (5) such that the beam of coherent light impinges upon a portion of the region on the plate (1) corresponding to the position on the plate of the target colony (20) and the central axis of the beam (23) impinges upon the plate (1) such that this point is centred upon the position on the plate of the target colony; and arranged to adjust a part of the region on the plate upon which the beam of coherent light impinges by adjusting either or both of: the direction of the beam; and the position of the plate and/or to adjust the relative positions of the light source (5) and the plate such that the beam is directed towards the position on the plate of the target colony
wherein computer is configured to:
- to control the mechanism so as to expose the target colony to the beam of coherent light by directing the beam (23) towards an optical centroid of the target colony (20);
- and the mechanism so as to direct the beam towards the optical centroid by adjusting the relative positions of the plate and the light source such that the beam coincides with the position on the plate of the target colony, and to correct the relative positions until the beam coincides with a point corresponding to a maximum value of the symmetry parameter;
- and the mechanism so as to direct the beam towards the optical centroid by iteratively adjusting the point within the region on the plate upon which the central axis of the beam impinges and generating a symmetry parameter, until a maximum value of the symmetry parameter is generated and a point corresponding to this value is located;
- use an image analysis algorithm to generate the symmetry parameter for a point within the region on the plate based upon any of the symmetry, contrast or homogeneity of the monitored diffraction pattern produced when the beam of coherent light is centred upon that point, and wherein the optical centroid is defined as a point within the target colony corresponding to the greatest value of the symmetry parameter.

11. A system according to any of claim 9 , wherein the system comprises an optical system comprising the coherent light source (5), and wherein the mechanism (19) is configured to expose the target colony (20) to the beam of coherent light (23) by adjusting the optical system such that the beam is directed towards the position on the plate (1) of the target colony or by adjusting the optical system relative to the plate by moving the plate in any of a direction Z parallel to the optical axis of the system and directions X and Y perpendicular to the optical axis of the system, preferably the system comprises a camera (2) configured to acquire data by acquiring an image of the region on the plate and arranged to record the image from a direction perpendicular, parallel or from a direction inclined at an angle of 45 degrees with respect to the surface of the plate upon which the at least one colony is disposed, and wherein the system is configured to acquire the data by exposing the region on the plate to radiation by either: directing a light source to irradiate the region; or exposing the region to ambient light, and acquiring an image of the region on the plate and the at least one colony of microorganisms using the camera and the image is recorded from the direction parallel to the surface of the plate by recording the image reflected from a light splitter (3).

12. A system according to claim 10 , comprising a mechanism (19) configured to cause the beam (23) of coherent light to scan the region on the plate (1) by adjusting the relative positions of the light source (5) and the plate so as to acquire the data; and the detector (14) configured to monitor light resulting from the beam of coherent light scanning the region on the plate and to detect light diffracted by the target colony so as to locate the position of the target colony.

13. A system according to claim 11 or 12, configured to execute the following method steps
- irradiate the at least one colony of microorganisms disposed on the plate;
- record, and send to a computer an image of the entire plate and the at least one colony of microorganisms;
- analyze the image using computer algorithms for processing visual information so as to distinguish the location of the at least one colony of microorganisms recorded; and then to: move the plate and the at least one colony of microorganisms relative to an optical system comprising a source of the coherent light beam;
- acquire an enlarged image of each of at least one individual colony;
- irradiate a single colony of microorganisms of the at least one colony of microorganisms with the coherent beam of light;
- and subsequent to monitoring the diffraction pattern of the target colony, record and compare the monitored pattern with a reference pattern; and applied the algorithms for processing visual information comprising the analysis of colony morphologies,
- the target colony data is generated by comparing the monitored diffraction pattern with the reference diffraction pattern and in order to identify or characterize microorganisms within the target colony
wherein the computer is configured to:
- control the performing of the step of acquiring data and is further configured to repeat this step at least until the acquired data indicates the position on the plate of the target colony;
- and to repeat the step at a predetermined interval and over a sufficiently long period of time for a colony of microorganisms to grow on the plate, and wherein the data indicating the position on the plate of the target colony is defined by the target colony having grown to such a size that it may be visually located in accordance with the data;
- and to control the performing of the steps of exposing the target colony to the beam, monitoring the diffraction pattern, and generating target colony data, and is further configured to repeat these steps at least until the target colony data includes an identified species present in the target colony;
- and to repeat the steps according to a predetermined time interval and over a sufficiently long period of time for a colony of microorganisms to grow on the plate, and wherein the target colony data including an identified species present in the target colony is defined by the target colony having grown to such a size that the diffraction pattern contains sufficient data to identify the species with reference to a reference diffraction pattern;
- and to generate target colony data in accordance with two or more monitored diffraction patterns, wherein the two or more patterns are monitored at different times and are representative of the diffractive properties of the target colony at the different times

14. A system according to claim 13 , wherein the computer is configured , to generate target colony data in accordance with two or more monitored diffraction patterns, wherein the two or more patterns are monitored at different times and are representative of the diffractive properties of the target colony at the different times; and wherein the target colony data is representative of a change in the diffractive properties of the target colony between the different times and wherein each of the inoculator device and the incubator comprises an automated system configured to inoculate and incubate the at least one microorganism in accordance with predetermined inoculation parameters and incubation parameters, which are configured such that, each time the method is performed, each of the inoculator device and the incubator perform the physical processes, by way of which the colonies of microorganisms are inoculated and incubated, identically
more preferably the inoculator device is configured to inoculate the at least one microorganism by disposing the microorganism on the plate, and wherein the computer is configured to acquire the data representing the region on the plate in accordance with the inoculation parameters, such that the data includes an indication of the portion of the plate within which the at least one microorganism was disposed; and wherein the computer is configured to locate the position on the plate of the target colony in accordance with the indicated portion of the plate, and wherein the mechanism is configured to direct the light source so as to cause the light beam to scan the plate along a path representing an inoculation track by which the at least one microorganism was inoculated onto the plate.

15. A system according to any of claims 9 to 14 , the system comprising:
- optical elements (6) for modulating a beam of light emitted by the coherent light source;
- a converging lens (11); a stand (8), the plate being disposed between the lens and a detector (14) for mounting the plate (1);
- a light splitter (3) disposed between the lens and the plate;
- a camera (2) comprising two lenses or a lens with a variable focal length situated obliquely in relation to the direction of propagation of the beam of light and beside the light splitter, and configured to record an image of the plate and/or enlarged images of at least one individual colony of microorganisms disposed on the plate,
- the camera being connected with the computer;
- the stand comprising the light source comprises a plurality of diodes arranged in the form of a ring at the edge of the plate and
- a drive for moving the light source,
- the computer being comprising algorithms for processing visual information.

16. A system according to claim 15 , wherein the detector (13) is a camera comprising two lenses or a lens with a variable focal length and the stand comprises a drive (9) for moving it in directions X and Y perpendicular to an optical axis of the system , the system further comprising a second drive (10) allowing for moving the coherent light source along with the element for modulating it and the lens, as a whole, in direction Z parallel to the optical axis of the system, wherein the stand comprises the source of radiating light and the drive for moving it in direction Z parallel to an optical axis of the system and directions X and Y perpendicular to the optical axis of the system, while the detector is a camera comprising at least two lenses and at least one of the lenses has a variable focal length, and the computer further comprises the algorithms for processing visual information.

17. A method according to claim 1, wherein the enlarged images of individual colonies are analyzed by algorithms for processing visual information to comprise analysis of colony morphological data, wherein the morphological data are used in combination with diffraction analysis data in order to accurately identify or characterize microorganisms within the target colony.

## Patentansprüche

1. Verfahren zur Identifizierung von Mikroorganismen, die beliebige von Bakterien, Archaeen, Eukaryonten, Protisten, Rädertierchen, Pilze und Hefezellen umfassen, wobei das Verfahren die automatische Durchführung der folgenden Schritte umfasst:
- Impfen wenigstens eines Mikroorganismus auf eine Platte; und Inkubieren des wenigstens einen Mikroorganismus, um die Entwicklung wenigstens einer Kolonie von Mikroorganismen zu ermöglichen, die in einem Bereich auf der Platte angeordnet sind, wobei sowohl das Impfen als auch das Inkubieren durch ein automatisiertes System in Übereinstimmung mit vorbestimmten Impfparametern bzw. Inkubationsparametern durchgeführt wird;
und weiterhin umfassend:
- Erfassen von Daten, die den Bereich auf der Platte darstellen, der ein Kulturmedium umfasst, wobei die wenigstens eine Kolonie von Mikroorganismen auf dem Medium angeordnet ist;
- Lokalisierung der Position einer Zielkolonie der wenigstens einen Kolonie von Mikroorganismen auf der Platte in Übereinstimmung mit den Daten;
- Erfassung von Daten zu verschiedenen Zeitpunkten, wobei die Daten jeweils ein vergrößertes Bild von wenigstens einer einzelnen Kolonie, die eine Zielkolonie ist, umfassen;
- Einwirkenlassen eines modulierten kohärenten Lichtstrahls auf die Zielkolonie entsprechend den Daten;
- Überwachen eines Beugungsmusters, das sich aus der Beugung des Lichts durch die Zielkolonie ergibt, zu verschiedenen Zeiten; und
- Erzeugen von Zielkoloniedaten zu verschiedenen Zeitpunkten, die eine in der Zielkolonie vorhandene Spezies in Übereinstimmung mit dem überwachten Beugungsmuster und einem Referenzbeugungsmuster anzeigen;
wobei die Zielkoloniedaten durch Vergleichen des überwachten Beugungsmusters mit dem Referenzbeugungsmuster erzeugt werden, umfassend das Erzeugen eines Vergleichsparameters auf der Grundlage eines Vergleichs des überwachten Beugungsmusters mit jedem einer Vielzahl von Referenzbeugungsmustern, wobei jedes Referenzbeugungsmuster einer vorbestimmten Spezies entspricht; und Identifizieren der in der Zielkolonie vorhandenen Zielspezies als die vorbestimmte Art, für die der erzeugte Vergleichsparameter den größten Wert hat, und bei der der modulierte kohärente Lichtstrahl so konfiguriert ist, dass er die Platte entlang eines Pfades abtastet, der eine Beimpfungsspur darstellt, mit dem der wenigstens eine Mikroorganismus auf die Platte geimpft wurde, wobei der modulierte kohärente Lichtstrahl ein Licht ist, das von einer Quelle (5) für kohärentes Licht erzeugt wird, das durch ein Modulationselement (6) moduliert wird, das optische Elemente umfasst, die den Durchmesser, die Intensität und die Wellenfront des Strahls des kohärenten Lichts aus der Quelle (5) verändern.

2. Verfahren gemäß Anspruch 1, bei dem ein Teil des Bereichs auf der Platte, auf den der Strahl kohärenten Lichts auftrifft, eingestellt wird, indem entweder die Richtung des modulierten kohärenten Strahls und/oder die Position der Platte eingestellt wird, was durch Richten des modulierten kohärenten Lichtstrahls in Richtung des optischen Schwerpunkts erreicht wird, der als ein Punkt innerhalb der Zielkolonie definiert ist, der dem größten Wert eines Symmetrieparameters entspricht, der sich auf die Symmetrie, den Kontrast oder die Homogenität des überwachten Beugungsmusters bezieht, so dass der modulierte kohärente Lichtstrahl mit der Position der Zielkolonie auf der Platte zusammenfällt, und die relativen Positionen korrigiert werden, bis der modulierte kohärente Lichtstrahl mit einem Punkt zusammenfällt, der einem maximalen Wert des Symmetrieparameters entspricht.

3. Verfahren gemäß Anspruch 2 oder 1, wobei das Erfassen der Daten das Erfassen eines Bilds des Bereichs auf der Platte durch Bestrahlung des Bereichs auf der Platte und Aufnahme eines Bildes des Bereichs und der wenigstens einen Kolonie von Mikroorganismen umfasst, wobei das Bild aus einer Richtung senkrecht zu demjenigen Oberflächenbereich der Platte aufgenommen wird, in dem sich die wenigstens eine Kolonie befindet, oder das Bild aus der Richtung parallel zur Oberfläche der Platte aufgezeichnet wird, indem der modulierte kohärente Lichtstrahl, der von einem Lichtteiler (3) reflektiert wird, aufgezeichnet wird und die Lokalisierung der Position auf die Platte (1) der Zielkolonie durch die Analyse des aufgenommenen Bildes mit Hilfe eines Computers (4) erreicht wird, der so konfiguriert ist, dass er Bilddaten verarbeiten kann, um eine Kolonie von Mikroorganismen auf dem Bild zu identifizieren und ihre Position zu lokalisieren.

4. Verfahren gemäß einem der vorhergehenden Ansprüche, wobei die Erfassung der Daten das Bewirken, dass der modulierte kohärente Lichtstrahl den Bereich auf der Platte (1) abtastet, umfasst, und das Lokalisieren der Position der Zielkolonie auf der Platte durch Überwachung des Lichts, das aus dem modulierten kohärenten Lichtstrahl, der den Bereich auf der Platte (4) abtastet, resultiert, und Erfassen des von der Zielkolonie gebeugten Lichts durchgeführt wird.

5. Verfahren gemäß einem der vorhergehenden Ansprüche, wobei vor der Überwachung des Beugungsmusters der Zielkolonie die wenigstens eine Kolonie von Mikroorganismen, die sich in dem Bereich auf der Platte befindet, bestrahlt wird;
ein Bild der gesamten Platte und der wenigstens einen Kolonie von Mikroorganismen aufgenommen, an einen Computer gesendet und mit Hilfe von Computeralgorithmen zur Verarbeitung visueller Daten analysiert wird und der Standort der wenigstens einen aufgezeichneten Kolonie von Mikroorganismen erkannt wird, und dann: die Platte und die wenigstens eine Kolonie von Mikroorganismen relativ zu einer Optik mit einer Quelle für den kohärenten Lichtstrahl bewegt wird; und
eine einzelne Kolonie von Mikroorganismen der wenigstens einen Kolonie von Mikroorganismen mit dem modulierten kohärenten Lichtstrahl bestrahlt wird.

6. Verfahren gemäß Anspruch 5, wobei die Algorithmen zur Verarbeitung visueller Daten die Analyse von Koloniemorphologien, die die Zielkoloniedaten, die sich auf die physischen oder scheinbaren Formen beziehen oder diese anzeigen, erweitern, umfassen und geometrische Eigenschaften der mikrobiellen Kolonien auf das vergrößerte Bild angewendet werden;
und in Kombination mit Beugungsanalysedaten verwendet werden, um Mikroorganismen in der Zielkolonie zu identifizieren oder zu charakterisieren;
wobei die Zielkoloniedaten durch Vergleich des überwachten Beugungsmusters mit dem Referenzbeugungsmuster erzeugt werden und vorzugsweise der Schritt des Erfassens der Daten wenigstens so lange wiederholt wird, bis die erfassten Daten die Position der Zielkolonie auf der Platte anzeigen, und das Bestrahlen der Zielkolonie, das Überwachen des Beugungsmusters und das Erzeugen von Zielkoloniedaten wenigstens so lange wiederholt werden, bis die Zielkoloniedaten eine identifizierte Spezies enthalten, die in der Zielkolonie vorhanden ist, und besonders bevorzugt die Schritte in einem vorbestimmten Zeitintervall und über einen ausreichend langen Zeitraum wiederholt werden, damit eine Kolonie von Mikroorganismen auf der Platte wachsen kann, und wobei die Daten, die die Position der Zielkolonie auf der Platte anzeigen, und die Daten der Zielkolonie, die eine identifizierte Spezies enthalten, die in der Zielkolonie vorhanden ist, dadurch definiert sind, dass die Zielkolonie eine solche Größe erreicht hat, dass sie in Übereinstimmung mit den Daten visuell lokalisiert werden kann, und das Beugungsmuster ausreichende Daten enthält, um die Spezies mit Bezug auf ein Referenzbeugungsmuster zu identifizieren.

7. Verfahren gemäß Anspruch 6, wobei die Daten der Zielkolonie in Übereinstimmung mit zwei oder mehr überwachten Beugungsmustern erzeugt werden, wobei die zwei oder mehreren Muster zu verschiedenen Zeitpunkten beobachtet werden und repräsentativ für die Beugungseigenschaften der Zielkolonie und deren Veränderungen zu den verschiedenen Zeitpunkten sind und die Impf- und Inkubationsparameter so konfiguriert sind, dass bei jeder Durchführung des Verfahrens die physikalischen Prozesse, mit denen die Mikroorganismenkolonien beimpft und inkubiert werden, identisch ablaufen.

8. Verfahren gemäß einem der Ansprüche 7, wobei die Daten, die die Region auf der Platte repräsentieren, in Übereinstimmung mit den Beimpfungsparametern erfasst werden, so dass die Daten eine Angabe des Teils der Platte enthalten, in dem sich der wenigstens eine Mikroorganismus befand, und die Position der Zielkolonie auf der Platte in Übereinstimmung mit dem angegebenen Teil der Platte lokalisiert wird oder der Lichtstrahl die Platte entlang eines Weges abtastet, der eine Beimpfungsspur darstellt, durch die der wenigstens eine Mikroorganismus auf die Platte geimpft wurde.

9. System zur Identifizierung von Mikroorganismen, wobei das System umfasst:
- eine Platte (1), auf der Kolonien von Mikroorganismen (17) angeordnet werden können;
- eine Impfvorrichtung zum Impfen wenigstens eines Mikroorganismus auf die Platte entlang einer Beimpfungsspur;
- einen Inkubator (22) zum Inkubieren des wenigstens einen Mikroorganismus, um die Entwicklung wenigstens einer Kolonie von Mikroorganismen auf der Platte zu ermöglichen;
- eine kohärente Lichtquelle (5), die einen kohärenten Lichtstrahl (23) aussendet;
- einen Mechanismus (19), der so konfiguriert ist, dass er einen Strahl kohärenten Lichts, der von der Lichtquelle emittiert wird, in Bezug auf die Platte lenkt;
- ein Modulationselement (6), das Elemente umfasst, die so konfiguriert sind, dass sie den Durchmesser, die Intensität und die Wellenfront des kohärenten Lichtstrahls (23) verändern, um einen modulierten kohärenten Lichtstrahl zu erzeugen;
- einen Computer (4), der so konfiguriert ist, dass er Daten, die einen Bereich auf einer Platte darstellen, innerhalb dessen sich wenigstens eine Kolonie von Mikroorganismen befindet und die die Position der Zielkolonie auf der Platte anzeigen, erfassen, die Position der Zielkolonie der wenigstens einen Kolonie von Mikroorganismen in Übereinstimmung mit den Daten auf der Platte lokalisieren und den Mechanismus (19) so steuern kann, dass die Zielkolonie dem Strahl ausgesetzt wird; und
- einen Detektor (14), der so konfiguriert ist, dass er zu verschiedenen Zeiten ein Beugungsmuster (16) überwacht, das aus der Beugung des Lichts an der Zielkolonie resultiert;
wobei der Computer weiterhin so konfiguriert ist, dass er Zielkoloniedaten erzeugt, die Angaben zu einer in der Zielkolonie vorhandenen Spezies in Übereinstimmung mit dem überwachten Beugungsmuster und einem Referenzbeugungsmuster umfassen;
und wobei der Detektor (14) weiterhin so konfiguriert ist, dass er Bilddaten, die ein Bild des Bereichs repräsentieren, in Reaktion auf die auf den Detektor auftreffende Strahlung erfassen kann, und wobei die Daten, die den Bereich auf der Platte darstellen, die Bilddaten umfassen; und wobei der Computer so konfiguriert ist, dass er die Position der Zielkolonie der wenigstens einen Kolonie von Mikroorganismen auf der Platte in Übereinstimmung mit den Bilddaten lokalisieren und auf der Grundlage eines Vergleichs des überwachten Beugungsmusters mit jedem einer Vielzahl von Referenzbeugungsmustern einen Vergleichsparameter erzeugen kann, wobei jedes Referenzbeugungsmuster einer vorbestimmten Spezies entspricht, und die in der Zielkolonie vorhandene Zielspezies als die vorbestimmte Spezies, für die der erzeugte Vergleichsparameter den größten Wert aufweist, identifizieren kann, und wobei das System so konfiguriert ist, dass es den modulierten kohärenten Lichtstrahl entlang eines Pfades, der die Beimpfungsspur darstellt, durch den der wenigstens eine Mikroorganismus auf die Platte geimpft wurde, abtasten lassen kann.

10. System gemäß Anspruch 9, wobei der Mechanismus (19), der Robotik-Aktoren umfasst, die so konfiguriert sind, dass sie die Platte (1) und die kohärente Lichtquelle (5) bewegen können, so konfiguriert ist, dass er die Zielkolonie (20) dem Strahl des kohärenten Lichts (23) aussetzen kann, indem er die Lichtquelle (5) so ausrichtet, dass der Strahl des kohärenten Lichts auf einen Teil des Bereichs auf der Platte (1) auftrifft, der der Position der Zielkolonie (20) auf der Platte entspricht, und die zentrale Achse des Strahls (23) so auf die Platte (1) auftrifft, dass dieser Punkt auf der Position der Zielkolonie auf der Platte zentriert ist; und so angeordnet ist, dass ein Teil des Bereichs auf der Platte, auf den der Strahl kohärenten Lichts auftrifft, durch Einstellen der Richtung des Strahls und/oder der Position der Platte eingestellt wird und/oder die relativen Positionen der Lichtquelle (5) und die Platte so eingestellt werden, dass der Strahl auf die Position der Zielkolonie auf der Platte gerichtet ist;
wobei der Computer so konfiguriert ist, dass er:
den Mechanismus so steuern kann, dass die Zielkolonie dem Strahl des kohärenten Lichts ausgesetzt wird, indem der Strahl (23) in Richtung eines optischen Schwerpunkts der Zielkolonie (20) gelenkt wird;
und den Mechanismus so steuern kann, dass der Strahl auf den optischen Schwerpunkt gerichtet wird, indem die relativen Positionen der Platte und der Lichtquelle so eingestellt werden, dass der Strahl mit der Position der Zielkolonie auf der Platte zusammenfällt, und die relativen Positionen zu korrigieren, bis der Strahl mit einem Punkt zusammenfällt, der einem Maximalwert des Symmetrieparameters entspricht;
und den Mechanismus so steuern kann, dass der Strahl auf den optischen Schwerpunkt gerichtet wird, indem der Punkt innerhalb des Bereichs auf der Platte, auf den die Mittelachse des Strahls auftrifft, iterativ eingestellt und ein Symmetrieparameter erzeugt wird, bis ein maximaler Wert des Symmetrieparameters ermittelt und ein Punkt, der diesem Wert entspricht, lokalisiert wird;
einen Bildanalysealgorithmus verwenden kann, um den Symmetrieparameter für einen Punkt innerhalb des Bereichs auf der Platte auf der Grundlage der Symmetrie, des Kontrasts oder der Homogenität des überwachten Beugungsmusters, das erzeugt wird, wenn der Strahl kohärenten Lichts auf diesen Punkt zentriert wird, zu erzeugen, und wobei der optische Schwerpunkt als ein Punkt innerhalb der Zielkolonie definiert ist, der dem größten Wert des Symmetrieparameters entspricht.

11. System gemäß einem der Ansprüche 9, wobei das System eine Optik umfasst, die die kohärente Lichtquelle (5) umfasst, und wobei der Mechanismus (19) so konfiguriert ist, dass er die Zielkolonie (20) dem Strahl des kohärenten Lichts (23) aussetzen kann, indem die Optik so eingestellt wird, dass der Strahl auf die Position der Zielkolonie auf der Platte (1) gerichtet wird, oder die Optik durch Bewegen der Platte in einer beliebigen Richtung Z parallel zur optischen Achse des Systems und in den Richtungen X und Y senkrecht zur optischen Achse des Systems relativ zur Platte eingestellt wird, wobei das System vorzugsweise eine Kamera (2) umfasst, die so konfiguriert ist, dass sie Daten erfassen kann, indem sie ein Bild der Region auf der Platte erfasst, und die so angeordnet ist, dass sie das Bild aus einer Richtung senkrecht, parallel oder aus einer Richtung, die in einem Winkel von 45 Grad in Bezug auf die Oberfläche der Platte, auf der sich die wenigstens eine Kolonie befindet, geneigt ist, aufzeichnen kann, und wobei das System so konfiguriert ist, dass es die Daten erfasst, indem es den Bereich auf der Platte einer Strahlung aussetzt, indem es entweder eine Lichtquelle so ausrichtet, dass sie den Bereich bestrahlt, oder indem es den Bereich Umgebungslicht aussetzt, und indem es ein Bild des Bereichs auf der Platte und der wenigstens einen Kolonie von Mikroorganismen unter Verwendung der Kamera aufnimmt, und das Bild aus der Richtung parallel zur Oberfläche der Platte durch Aufnahme des von einem Lichtteiler (3) reflektierten Bildes aufgezeichnet wird.

12. System gemäß Anspruch 10, umfassend einen Mechanismus (19), der so konfiguriert ist, dass er bewirkt, dass der Strahl (23) von kohärentem Licht den Bereich auf der Platte (1) durch Einstellen der relativen Positionen der Lichtquelle (5) und der Platte abtastet, um die Daten zu erfassen; und der Detektor (14) so konfiguriert ist, dass er Licht, das aus dem Strahl kohärenten Lichts resultiert, der den Bereich auf der Platte abtastet, überwachen kann und das von der Zielkolonie gebeugte Licht nachweisen kann, um die Position der Zielkolonie zu lokalisieren.

13. System gemäß Anspruch 11 oder 12, das so konfiguriert ist, dass es die folgenden Verfahrensschritte ausführen kann:
- Bestrahlung der wenigstens einen Kolonie von Mikroorganismen, die sich auf der Platte befindet;
- ein Bild der gesamten Platte und der wenigstens einen Kolonie von Mikroorganismen aufzeichnen und an einen Computer senden;
- das Bild mit Hilfe von Computeralgorithmen zur Verarbeitung visueller Daten analysieren, um den Ort der wenigstens einen aufgezeichneten Kolonie von Mikroorganismen zu kennzeichnen; und dann: die Platte und die wenigstens eine Kolonie von Mikroorganismen relativ zu einer Optik, die eine Quelle für den kohärenten Lichtstrahl umfasst, zu bewegen;
- ein vergrößertes Bild von wenigstens einer einzelnen Kolonie aufnehmen;
- Bestrahlen einer einzelnen Kolonie von Mikroorganismen der wenigstens einen Kolonie von Mikroorganismen mit dem kohärenten Lichtstrahl;
- und im Anschluss an die Überwachung des Beugungsmusters der Zielkolonie das überwachte Muster aufzeichnen und mit einem Referenzmuster vergleichen; und die Algorithmen zur Verarbeitung visueller Daten, die die Analyse der Koloniemorphologien umfassen, anwenden;
- die Daten der Zielkolonie durch den Vergleich des überwachten Beugungsmusters mit dem Referenzbeugungsmuster erzeugt werden, und um Mikroorganismen innerhalb der Zielkolonie zu identifizieren oder zu charakterisieren,
wobei der Computer so konfiguriert ist, dass er:
- die Durchführung des Schritts der Datenerfassung steuert, und weiterhin so konfiguriert ist, dass er diesen Schritt zumindest solange wiederholt, bis die erfassten Daten die Position der Zielkolonie auf der Platte anzeigen;
- und den Schritt in einem vorbestimmten Intervall und über einen ausreichend langen Zeitraum wiederholen, damit eine Kolonie von Mikroorganismen auf der Platte wachsen kann, und wobei die Daten, die die Position der Zielkolonie auf der Platte anzeigen, dadurch definiert sind, dass die Zielkolonie eine solche Größe erreicht hat, dass sie in Übereinstimmung mit den Daten visuell lokalisiert werden kann;
- und das Durchführen der Schritte des Einwirkenlassens des Strahls auf die Zielkolonie, des Überwachens des Beugungsmusters und des Erzeugens von Zielkoloniedaten steuern, und ist weiterhin so konfiguriert, dass er diese Schritte wenigstens solange wiederholt, bis die Zielkoloniedaten eine identifizierte Spezies enthalten, die in der Zielkolonie vorhanden ist;
- und wiederholen der Schritte in einem vorbestimmten Zeitintervall und über einen ausreichend langen Zeitraum, damit eine Kolonie von Mikroorganismen auf der Platte wächst, und wobei die Zielkoloniedaten, die eine in der Zielkolonie vorhandene identifizierte Spezies enthalten, dadurch definiert sind, dass die Zielkolonie eine solche Größe erreicht hat, dass das Beugungsmuster ausreichende Daten enthält, um die Spezies mit Bezug auf ein Referenzbeugungsmuster zu identifizieren;
- und Erzeugen von Zielkoloniedaten in Übereinstimmung mit zwei oder mehr überwachten Beugungsmustern, wobei die zwei oder mehr Muster zu verschiedenen Zeiten überwacht werden und für die Beugungseigenschaften der Zielkolonie zu den verschiedenen Zeiten repräsentativ sind.

14. System gemäß Anspruch 13, wobei der Computer so konfiguriert ist, dass er Zielkoloniedaten in Übereinstimmung mit zwei oder mehr überwachten Beugungsmustern erzeugen kann, wobei die zwei oder mehr Muster zu verschiedenen Zeiten überwacht werden und repräsentativ für die Beugungseigenschaften der Zielkolonie zu den verschiedenen Zeiten sind; und wobei die Zielkoloniedaten für eine Änderung der Beugungseigenschaften der Zielkolonie zwischen den verschiedenen Zeitpunkten repräsentativ sind und wobei sowohl die Impfvorrichtung als auch der Inkubator ein automatisiertes System umfassen, das so konfiguriert ist, dass es den wenigstens einen Mikroorganismus in Übereinstimmung mit vorbestimmten Impfparametern und Inkubationsparametern impft und inkubiert, die so konfiguriert sind, dass jedes Mal, wenn das Verfahren durchgeführt wird, sowohl die Impfvorrichtung als auch der Inkubator die physikalischen Prozesse, durch die die Kolonien von Mikroorganismen geimpft und inkubiert werden, identisch durchführen,
besonders bevorzugt die Impfvorrichtung so konfiguriert ist, dass sie den wenigstens einen Mikroorganismus durch Anordnen des Mikroorganismus auf der Platte impft, und wobei der Computer so konfiguriert ist, dass er die Daten, die den Bereich auf der Platte repräsentieren, in Übereinstimmung mit den Impfparametern erfasst, so dass die Daten eine Angabe des Teils der Platte enthalten, in dem der wenigstens eine Mikroorganismus angeordnet wurde; und wobei der Computer so konfiguriert ist, dass er die Position der Zielkolonie auf der Platte in Übereinstimmung mit dem angegebenen Teil der Platte lokalisiert, und wobei der Mechanismus so konfiguriert ist, dass er die Lichtquelle so ausrichtet, dass der Lichtstrahl die Platte entlang eines Pfades abtastet, der eine Beimpfungsspur darstellt, durch die der wenigstens eine Mikroorganismus auf die Platte geimpft wurde.

15. System gemäß einem der Ansprüche 9 bis 14, wobei das System umfasst:
- optische Elemente (6) zur Modulation eines von der kohärenten Lichtquelle ausgesandten Lichtstrahls;
- eine Sammellinse (11);
- einen Ständer (8), wobei die Platte zwischen der Linse und einem Detektor (14) zur Befestigung der Platte (1) angeordnet ist;
- einen Lichtteiler (3), der zwischen der Linse und der Platte angeordnet ist;
- eine Kamera (2) mit zwei Linsen oder einer Linse mit variabler Brennweite, die schräg zur Ausbreitungsrichtung des Lichtstrahls und neben dem Lichtteiler angeordnet ist und so konfiguriert ist, dass sie ein Bild der Platte und/oder vergrößerte Bilder von wenigstens einer einzelnen Kolonie von Mikroorganismen, die auf der Platte angeordnet sind, aufnehmen kann;
- wobei die Kamera mit dem Computer verbunden ist;
- der Ständer mit der Lichtquelle eine Vielzahl von Dioden umfasst, die in Form eines Rings am Rand der Platte angeordnet sind; und
- einen Antrieb für die Bewegung der Lichtquelle;
- wobei der Computer Algorithmen zur Verarbeitung visueller Daten umfasst.

16. System gemäß Anspruch 15, wobei der Detektor (13) eine Kamera ist, die zwei Linsen oder eine Linse mit veränderlicher Brennweite umfasst, und der Ständer über einen Antrieb (9) verfügt, um ihn in die Richtungen X und Y senkrecht zu einer optischen Achse des Systems zu bewegen, wobei das System weiterhin einen zweiten Antrieb (10) umfasst, der es ermöglicht, die kohärente Lichtquelle zusammen mit dem Element zu ihrer Modulation und der Linse als Ganzes in Richtung Z parallel zur optischen Achse des Systems zu bewegen, wobei der Ständer die Lichtquelle und den Antrieb zu ihrer Bewegung in Richtung Z parallel zu einer optischen Achse des Systems und in Richtung X und Y senkrecht zur optischen Achse des Systems umfasst, während der Detektor eine Kamera ist, die wenigstens zwei Linsen umfasst und wenigstens eine der Linsen eine variable Brennweite hat, und der Computer weiterhin die Algorithmen zur Verarbeitung visueller Daten umfasst.

17. Verfahren gemäß Anspruch 1, wobei die vergrößerten Bilder der einzelnen Kolonien durch Algorithmen zur Verarbeitung visueller Daten analysiert werden, wobei diese eine Analyse der morphologischen Daten der Kolonie umfassen, wobei die morphologischen Daten in Kombination mit Daten der Beugungsanalyse verwendet werden, um Mikroorganismen innerhalb der Zielkolonie genau zu identifizieren oder zu charakterisieren.

## Revendications

1. Procédé d'identification de micro-organismes comprenant l'un quelconque parmi des bactéries, des archées, des eucaryotes, des protistes, des rotifères, des champignons et des cellules de levure, le procédé comprenant la réalisation automatique des étapes suivantes :
- l'inoculation d'au moins un micro-organisme sur une plaque ; et l'incubation de l'au moins un micro-organisme de façon à permettre le développement d'au moins une colonie de micro-organismes disposée dans une région sur la plaque, dans lequel chacune de l'inoculation et de l'incubation est réalisée par un système automatisé en conformité avec des paramètres d'inoculation et des paramètres d'incubation prédéterminés, respectivement
et comprenant en outre :
- l'acquisition de données représentant la région sur la plaque comprenant un milieu de culture, et l'au moins une colonie de micro-organismes est disposée sur le milieu ;
- la localisation de la position sur la plaque d'une colonie cible de l'au moins une colonie de micro-organismes, en conformité avec les données ;
- l'acquisition à différents moments de données comprenant une image agrandie de chacune d'au moins une colonie individuelle comme étant la colonie cible ;
- l'exposition de la colonie cible à un faisceau de lumière cohérente modulée, en conformité avec les données ;
- la surveillance à différents moments d'une figure de diffraction résultant de la diffraction de la lumière par la colonie cible ; et
- la génération de données de colonie cible à différents moments indiquant une espèce présente dans la colonie cible en conformité avec la figure de diffraction surveillée et une figure de diffraction de référence
dans lequel les données de colonie cible sont générées en comparant la figure de diffraction surveillée avec la figure de diffraction de référence, ce qui comprend la génération d'un paramètre de comparaison basé sur une comparaison de la figure de diffraction surveillée avec chacun d'une pluralité de figures de diffraction de référence, chaque figure de diffraction de référence correspondant à une espèce prédéterminée ; et en identifiant l'espèce cible présente dans la colonie cible en tant qu'espèce prédéterminée pour laquelle le paramètre de comparaison généré a la valeur la plus élevée et dans lequel le faisceau de lumière cohérente modulée est configuré pour balayer la plaque le long d'une trajectoire représentant une piste d'inoculation par laquelle l'au moins un micro-organisme a été inoculé sur la plaque, dans lequel le faisceau de lumière cohérente modulée est une lumière générée par une source (5) de lumière cohérente modulée par un élément modulant (6), qui comprend des éléments optiques qui changent le diamètre, l'intensité et le front d'onde du faisceau de la lumière cohérente provenant de la source (5).

2. Procédé selon la revendication 1, dans lequel une partie de la région sur la plaque sur laquelle le faisceau de lumière cohérente frappe est ajustée en ajustant l'un ou l'autre ou les deux parmi : la direction du faisceau cohérent modulé ; et la position de la plaque, ce qui est obtenu en dirigeant le faisceau de lumière cohérente modulée vers un centroïde optique défini comme un point au sein de la colonie cible correspondant à la plus grande valeur d'un paramètre de symétrie lié à la symétrie, au contraste ou à l'homogénéité de la figure de diffraction surveillée, de telle sorte que le faisceau de lumière cohérente modulée coïncide avec la position sur la plaque de la colonie cible, et en corrigeant les positions relatives jusqu'à ce que le faisceau de lumière cohérente modulée coïncide avec un point correspondant à une valeur maximale du paramètre de symétrie.

3. Procédé selon la revendication 2 ou 1, dans lequel l'acquisition des données comprend l'acquisition d'une image de la région sur la plaque en exposant la région sur la plaque à un rayonnement et en acquérant une image de la région et de l'au moins une colonie de micro-organismes, dans lequel l'image est enregistrée à partir d'une direction perpendiculaire à la région de surface de la plaque au sein de laquelle l'au moins une colonie est disposée ou l'image est enregistrée à partir de la direction parallèle à la surface de la plaque en enregistrant le faisceau de lumière cohérente modulée réfléchi par un séparateur de lumière (3), et la localisation de la position sur la plaque (1) de la colonie cible est obtenue en analysant l'image acquise à l'aide d'un ordinateur (4) configuré pour traiter des données d'image de façon à identifier et à localiser la position d'une colonie de micro-organismes au sein de l'image.

4. Procédé selon l'une quelconque des revendications précédentes, dans lequel l'acquisition des données comprend le fait d'amener le faisceau de lumière cohérente modulée à balayer la région sur la plaque (1) et la localisation de la position sur la plaque de la colonie cible est réalisée en surveillant une lumière résultant du faisceau de lumière cohérente modulée balayant la région sur la plaque (4) et en détectant une lumière diffractée par la colonie cible.

5. Procédé selon l'une quelconque des revendications précédentes, dans lequel, avant la surveillance de la figure de diffraction de la colonie cible :
l'au moins une colonie de micro-organismes disposée au sein de la région sur la plaque est irradiée ;
une image de la plaque entière et de l'au moins une colonie de micro-organismes est enregistrée, envoyée à un ordinateur, analysée à l'aide d'algorithmes informatiques de traitement d'informations visuelles, et la localisation de l'au moins une colonie de micro-organismes enregistrée est distinguée, puis :
la plaque et l'au moins une colonie de micro-organismes sont déplacées par rapport à un système optique comprenant une source du faisceau de lumière cohérente ; et
une seule colonie de micro-organismes de l'au moins une colonie de micro-organismes est irradiée avec le faisceau de lumière cohérente modulée.

6. Procédé selon la revendication 5, dans lequel les algorithmes de traitement d'informations visuelles comprenant une analyse de morphologies de colonies étendant les données de colonie cible liées à ou indiquant les formes physiques ou apparentes et les propriétés géométriques de colonies microbiennes sont appliqués à l'image agrandie ;
et utilisés en combinaison avec des données d'analyse par diffraction afin d'identifier ou de caractériser des micro-organismes au sein de la colonie cible ;
dans lequel les données de colonie cible sont générées en comparant la figure de diffraction surveillée avec la figure de diffraction de référence et, de préférence,
l'étape d'acquisition des données est répétée au moins jusqu'à ce que les données acquises indiquent la position sur la plaque de la colonie cible et l'exposition de la colonie cible au faisceau, la surveillance de la figure de diffraction et la génération des données de la colonie cible sont répétées au moins jusqu'à ce que les données de colonie cible incluent une espèce identifiée présente dans la colonie cible et de manière davantage préférée
les étapes sont répétées selon un intervalle de temps prédéterminé et sur une période suffisamment longue pour qu'une colonie de micro-organismes croisse sur la plaque,
et dans lequel les données indiquant la position de la colonie cible sur la plaque et les données de colonie cible incluant une espèce identifiée présente dans la colonie cible sont définies par le fait que la colonie cible a crû jusqu'à une taille telle qu'elle peut être localisée visuellement en conformité avec les données et que la figure de diffraction contient suffisamment de données pour identifier l'espèce en référence à une figure de diffraction de référence.

7. Procédé selon la revendication 6, dans lequel les données de colonie cible sont générées en conformité avec deux figures de diffraction surveillées ou plus, dans lequel les deux figures ou plus sont surveillées à des moments différents et sont représentatives des propriétés de diffraction de la colonie cible et de leurs changements aux différents moments,
et des paramètres d'inoculation et d'incubation sont configurés de telle sorte que, chaque fois que le procédé est réalisé, les processus physiques par lesquels les colonies de micro-organismes sont inoculées et incubées sont réalisés de manière identique.

8. Procédé selon l'une quelconque des revendications 7, dans lequel les données représentant la région sur la plaque sont acquises en conformité avec les paramètres d'inoculation, de telle sorte que les données incluent une indication de la portion de la plaque au sein de laquelle l'au moins un micro-organisme a été disposé ; et la position sur la plaque de la colonie cible est localisée en conformité avec la portion indiquée de la plaque ou le faisceau de lumière balaye la plaque le long d'une trajectoire représentant une piste d'inoculation par laquelle l'au moins un micro-organisme a été inoculé sur la plaque.

9. Système d'identification de micro-organismes, le système comprenant :
- une plaque (1) sur laquelle des colonies de micro-organismes (17) peuvent être disposées ;
- un dispositif d'inoculation pour inoculer au moins un micro-organisme sur la plaque le long d'une piste d'inoculation ;
- un incubateur (22) pour incuber l'au moins un micro-organisme de façon à permettre le développement de l'au moins une colonie de micro-organismes disposée sur la plaque ;
- une source de lumière cohérente (5) émettant un faisceau de lumière cohérente (23) ;
- un mécanisme (19) configuré pour diriger un faisceau de lumière cohérente émis par la source de lumière par rapport à la plaque ; un élément modulant (6) qui comprend des éléments configurés pour changer le diamètre, l'intensité et le front d'onde du faisceau de lumière cohérente (23) afin de générer un faisceau de lumière cohérente modulée ;
- un ordinateur (4) configuré pour acquérir des données représentant une région sur une plaque au sein de laquelle au moins une colonie de micro-organismes est disposée et indiquant la position sur la plaque de la colonie cible, localiser la position sur la plaque de la colonie cible de l'au moins une colonie de micro-organismes en conformité avec les données, et commander le mécanisme (19) de façon à exposer la colonie cible au faisceau, et
- un détecteur (14) configuré pour surveiller à différents moments une figure de diffraction (16) résultant de la diffraction de la lumière par la colonie cible ;
dans lequel l'ordinateur est en outre configuré pour générer des données de colonie cible, comprenant l'indication d'une espèce présente dans la colonie cible en conformité avec la figure de diffraction surveillée et une figure de diffraction de référence ;
et dans lequel le détecteur (14) est en outre configuré pour acquérir des données d'image représentant une image de la région en réponse à un rayonnement incident sur le détecteur, et dans lequel les données représentant la région sur la plaque comprennent les données d'image ;
et dans lequel l'ordinateur est configuré pour localiser la position sur la plaque de la colonie cible de l'au moins une colonie de micro-organismes en conformité avec les données d'image, et pour générer un paramètre de comparaison basé sur une comparaison de la figure de diffraction surveillée avec chacune d'une pluralité de figures de diffraction de référence, chaque figure de diffraction de référence correspondant à une espèce prédéterminée ; et pour identifier l'espèce cible présente dans la colonie cible comme l'espèce prédéterminée pour laquelle le paramètre de comparaison généré a la plus grande valeur et dans lequel le système est configuré pour balayer le faisceau de lumière cohérente modulée le long d'une trajectoire représentant la piste d'inoculation par laquelle l'au moins un micro-organisme a été inoculé sur la plaque.

10. Système selon la revendication 9, dans lequel le mécanisme (19), comprenant des actionneurs robotiques configurés pour déplacer la plaque (1) et la source de lumière cohérente (5), est configuré pour exposer la colonie cible (20) au faisceau de lumière cohérente (23) en dirigeant la source de lumière (5) de telle sorte que le faisceau de lumière cohérente frappe une portion de la région sur la plaque (1) correspondant à la position sur la plaque de la colonie cible (20) et que l'axe central du faisceau (23) frappe la plaque (1) de telle sorte que ce point est centré sur la position sur la plaque de la colonie cible ; et agencé pour ajuster une partie de la région sur la plaque sur laquelle le faisceau de lumière cohérente frappe en ajustant l'un ou l'autre ou les deux parmi : la direction du faisceau ; et la position de la plaque et/ou pour ajuster les positions relatives de la source de lumière (5) et de la plaque de telle sorte que le faisceau est dirigé vers la position sur la plaque de la colonie cible
dans lequel l'ordinateur est configuré pour :
- commander le mécanisme de façon à exposer la colonie cible au faisceau de lumière cohérente en dirigeant le faisceau (23) vers un centroïde optique de la colonie cible (20) ;
- et le mécanisme de façon à diriger le faisceau vers le centroïde optique en ajustant les positions relatives de la plaque et de la source de lumière de telle sorte que le faisceau coïncide avec la position sur la plaque de la colonie cible, et à corriger les positions relatives jusqu'à ce que le faisceau coïncide avec un point correspondant à une valeur maximale du paramètre de symétrie ;
- et le mécanisme de façon à diriger le faisceau vers le centroïde optique en ajustant itérativement le point au sein de la région sur la plaque sur laquelle l'axe central du faisceau frappe et en générant un paramètre de symétrie, jusqu'à ce qu'une valeur maximale du paramètre de symétrie soit générée et qu'un point correspondant à cette valeur soit localisé ;
- utiliser un algorithme d'analyse d'image pour générer le paramètre de symétrie pour un point au sein de la région sur la plaque basé sur l'un quelconque parmi la symétrie, le contraste ou l'homogénéité de la figure de diffraction surveillée produite lorsque le faisceau de lumière cohérente est centré sur ce point, et dans lequel le centroïde optique est défini comme un point au sein de la colonie cible correspondant à la plus grande valeur du paramètre de symétrie.

11. Système selon l'une quelconque des revendications 9, le système comprenant un système optique comprenant la source de lumière cohérente (5),
et dans lequel le mécanisme (19) est configuré pour exposer la colonie cible (20) au faisceau de lumière cohérente (23) en ajustant le système optique de telle sorte que le faisceau est dirigé vers la position sur la plaque (1) de la colonie cible ou
en ajustant le système optique par rapport à la plaque en déplaçant la plaque dans l'une quelconque parmi une direction Z parallèle à l'axe optique du système et des directions X et Y perpendiculaires à l'axe optique du système, de préférence le système comprend une caméra (2) configurée pour acquérir des données en acquérant une image de la région sur la plaque et agencée pour enregistrer l'image à partir d'une direction perpendiculaire, parallèle ou à partir d'une direction inclinée selon un angle de 45 degrés par rapport à la surface de la plaque sur laquelle l'au moins une colonie est disposée, et dans lequel le système est configuré pour acquérir les données en exposant la région sur la plaque à un rayonnement soit : en dirigeant une source de lumière pour irradier la région ; soit en exposant la région à la lumière ambiante, et en acquérant une image de la région sur la plaque et de l'au moins une colonie de micro-organismes à l'aide de la caméra et l'image est enregistrée à partir de la direction parallèle à la surface de la plaque en enregistrant l'image réfléchie par un séparateur de lumière (3).

12. Système selon la revendication 10, comprenant un mécanisme (19), configuré pour amener le faisceau (23) de lumière cohérente à balayer la région sur la plaque (1) en ajustant les positions relatives de la source de lumière (5) et de la plaque de façon à acquérir les données ; et le détecteur (14) configuré pour surveiller une lumière résultant du faisceau de lumière cohérente balayant la région sur la plaque et pour détecter une lumière diffractée par la colonie cible de façon à localiser la position de la colonie cible.

13. Système selon la revendication 11 ou 12, configuré pour exécuter les étapes de procédé suivantes :
- l'irradiation de l'au moins une colonie de micro-organismes disposée sur la plaque ;
- l'enregistrement, et l'envoi à un ordinateur, d'une image de la plaque entière et de l'au moins une colonie de micro-organismes ;
- l'analyse de l'image à l'aide d'algorithmes informatiques de traitement d'informations visuelles de façon à distinguer la localisation de l'au moins une colonie de micro-organismes enregistrée ; puis de façon à : déplacer la plaque et l'au moins une colonie de micro-organismes par rapport à un système optique comprenant une source du faisceau de lumière cohérente ;
- l'acquisition d'une image agrandie de chacune d'au moins une colonie individuelle ;
- l'irradiation d'une seule colonie de micro-organismes de l'au moins une colonie de micro-organismes avec le faisceau de lumière cohérent ;
- et après la surveillance de la figure de diffraction de la colonie cible, l'enregistrement et la comparaison de la figure surveillée avec une figure de référence ; et l'application des algorithmes de traitement d'informations visuelles comprenant l'analyse de morphologies de colonies,
- les données de colonie cible sont générées en comparant la figure de diffraction surveillée avec la figure de diffraction de référence et afin d'identifier ou de caractériser des micro-organismes au sein de la colonie cible
dans lequel l'ordinateur est configuré pour :
- commander la réalisation de l'étape d'acquisition des données et est en outre configuré pour répéter cette étape au moins jusqu'à ce que les données acquises indiquent la position sur la plaque de la colonie cible ;
- et répéter l'étape à un intervalle prédéterminé et sur une période suffisamment longue pour qu'une colonie de micro-organismes croisse sur la plaque, et dans lequel les données indiquant la position sur la plaque de la colonie cible sont définies par le fait que la colonie cible a crû jusqu'à une taille telle qu'elle peut être localisée visuellement en conformité avec les données ;
- et commander la réalisation des étapes d'exposition de la colonie cible au faisceau, de surveillance de la figure de diffraction et de génération de données de colonie cible, et est en outre configuré pour répéter ces étapes au moins jusqu'à ce que les données de colonie cible incluent une espèce identifiée présente dans la colonie cible ;
- et répéter les étapes selon un intervalle de temps prédéterminé et sur une période suffisamment longue pour qu'une colonie de micro-organismes croisse sur la plaque, et dans lequel les données de colonie cible incluant une espèce identifiée présente dans la colonie cible sont définies par le fait que la colonie cible a crû jusqu'à une taille telle que la figure de diffraction contienne suffisamment de données pour identifier l'espèce en référence à une figure de diffraction de référence ;
- et générer des données de colonie cible en conformité avec deux figures de diffraction surveillées ou plus, dans lequel les deux figures ou plus sont surveillées à des moments différents et sont représentatives des propriétés de diffraction de la colonie cible aux différents moments.

14. Système selon la revendication 13, dans lequel l'ordinateur est configuré pour générer des données de colonie cible en conformité avec deux figures de diffraction surveillées ou plus, dans lequel les deux figures ou plus sont surveillées à des moments différents et sont représentatives des propriétés de diffraction de la colonie cible aux moments différents ; et dans lequel les données de colonie cible sont représentatives d'un changement des propriétés de diffraction de la colonie cible entre les différents moments et dans lequel chacun du dispositif d'inoculation et de l'incubateur comprend un système automatisé configuré pour inoculer et incuber l'au moins un micro-organisme en conformité avec des paramètres d'inoculation et des paramètres d'incubation prédéterminés, qui sont configurés de telle sorte que, chaque fois que le procédé est réalisé, chacun du dispositif d'inoculation et de l'incubateur réalise les processus physiques, par lesquels les colonies de micro-organismes sont inoculées et incubées, de manière identique
de manière davantage préférée, le dispositif d'inoculation est configuré pour inoculer l'au moins un micro-organisme en disposant le micro-organisme sur la plaque, et dans lequel l'ordinateur est configuré pour acquérir les données représentant la région sur la plaque en conformité avec les paramètres d'inoculation, de telle sorte que les données incluent une indication de la portion de la plaque au sein de laquelle l'au moins un micro-organisme a été disposé ; et dans lequel l'ordinateur est configuré pour localiser la position sur la plaque de la colonie cible en conformité avec la portion indiquée de la plaque, et dans lequel le mécanisme est configuré pour diriger la source de lumière de façon à amener le faisceau de lumière à balayer la plaque le long d'une trajectoire représentant une piste d'inoculation par laquelle l'au moins un micro-organisme a été inoculé sur la plaque.

15. Système selon l'une quelconque des revendications 9 à 14, le système comprenant :
- des éléments optiques (6) pour moduler un faisceau de lumière émis par la source de lumière cohérente ;
- une lentille convergente (11) ;
un support (8),
la plaque étant disposée entre la lentille et un détecteur (14) pour le montage de la plaque (1) ;
- un séparateur de lumière (3) disposé entre la lentille et la plaque ;
- une caméra (2) comprenant deux objectifs ou un objectif à focale variable située obliquement par rapport à la direction de propagation du faisceau de lumière et à côté du séparateur de lumière, et configurée pour enregistrer une image de la plaque et/ou des images agrandies d'au moins une colonie individuelle de micro-organismes disposée sur la plaque,
- la caméra étant connectée à l'ordinateur ;
- le support comprenant la source de lumière comprend une pluralité de diodes agencées sous forme d'anneau sur le bord de la plaque et
- un dispositif d'entraînement pour déplacer la source de lumière,
- l'ordinateur comprenant des algorithmes de traitement d'informations visuelles.

16. Système selon la revendication 15,
dans lequel le détecteur (13) est une caméra comprenant deux objectifs ou un objectif à focale variable et le support comprend un dispositif d'entraînement (9) pour le déplacer dans des directions X et Y perpendiculaires à un axe optique du système, le système comprenant en outre un second dispositif d'entraînement (10) permettant de déplacer la source de lumière cohérente ainsi que l'élément pour la moduler et l'objectif, dans son ensemble, dans la direction Z parallèle à l'axe optique du système,
dans lequel le support comprend la source de lumière rayonnante et le dispositif d'entraînement pour la déplacer dans une direction Z parallèle à un axe optique du système et dans des directions X et Y perpendiculaires à l'axe optique du système, tandis que le détecteur est une caméra comprenant au moins deux objectifs et au moins un des objectifs a une focale variable, et l'ordinateur comprend en outre les algorithmes de traitement d'informations visuelles.

17. Procédé selon la revendication 1, dans lequel les images agrandies de colonies individuelles sont analysées par des algorithmes de traitement d'informations visuelles pour comprendre une analyse des données morphologiques de colonie, dans lequel les données morphologiques sont utilisées en combinaison avec des données d'analyse de diffraction afin d'identifier ou de caractériser avec précision des micro-organismes au sein de la colonie cible.
